# EUROPÄISCHE PATENTSCHRIFT

(11) **EP 3 542 759 B1**
(45) Veröffentlichungstag und Bekanntmachung des Hinweises auf die Patenterteilung: **05.05.2021**
(21) Anmeldenummer: 19163778.4
(22) Anmeldetag: 19.03.2019
(51) Int. Cl.: A61F 2/32, A61F 2/46, A61F 2/36

(54) **FEMORALER HÜFTGELENKSPACER MIT SPÜLVORRICHTUNG**
FEMORAL HIP JOINT SPACER WITH FLUSHING DEVICE
ESPACEUR FÉMORALE DE L'ARTICULATION DE LA HANCHE POURVU DE DISPOSITIF DE RINÇAGE

(30) Priorität: 21.03.2018 DE 102018106705
(43) Veröffentlichungstag der Anmeldung: 25.09.2019
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- WO-A1-2017/178951
- US-A1- 2011 015 754
- US-A1- 2013 211 369
- US-A1- 2018 028 320

## Beschreibung

Die Erfindung betrifft einen femoralen Hüftgelenkspacer zum zeitweisen Ersetzen zumindest eines Teils eines Hüftgelenks, der für die Interimsphase von zweizeitigen septischen Revisionen von Hüftgelenk-Endoprothesen bestimmt ist, wie in den Ansprüchen definiert. Der Hüftgelenkspacer kann besonders bei solchen zweizeitigen septischen Revisionen Anwendung finden, bei denen zwei oder mehrere mikrobielle Keime ursächlich für eine Infektion der Hüftgelenk-Endoprothese und des umgebenden Gewebes sind.

Hüftgelenk-Endoprothesen werden in großem Umfang weltweit implantiert. Leider kommt es in einem geringen Prozentsatz vor, dass Hüftgelenk-Endoprothesen von mikrobiellen Keimen, besonders Gram-positiven Bakterien als auch Gram-negativen Bakterien und in sehr geringem Umfang von Hefen und Pilzen, besiedelt werden. Diese mikrobiellen Keime, hauptsächlich typische Hautkeime wie Staphylococcus aureus und Staphylococcus epidermidis, können während einer chirurgischen Operation (OP) in den Patienten gelangen. Daneben ist es auch möglich, dass mikrobielle Keime hämatogen Hüftgelenk-Endoprothesen erreichen. Bei einer Besiedlung von Hüftgelenk-Endoprothesen mit mikrobiellen Keimen wird das umliegende Knochen- und Weichgewebe mit infiziert und von den mikrobiellen Keimen geschädigt.

Stand der Technik sind vor allem zwei Behandlungsverfahren für infizierte Hüftgelenk-Endoprothesen, die einzeitige septische Revision und die zweizeitige septische Revision. Daneben gibt es noch eine Anzahl weiterer Behandlungsverfahren, wie zum Beispiel die Anwendung von Saug-Spül-Drainagen.

Bei der einzeitigen Revision wird innerhalb einer OP zuerst die infizierte Hüftgelenk-Endoprothesen entfernt, anschließend radikal debridiert und dann wird eine Revisions-Hüftgelenk-Endoprothese implantiert.

Bei zweizeitigen septischen Revisionen wird in einer ersten OP zuerst die infizierte Hüftgelenk-Endoprothesen entfernt, dann wird debridiert und anschließend erfolgt die Implantation eines Hüftgelenkspacers. Der Hüftgelenkspacer besteht aus einem Schaft, einem Kragen, einem Hals und einem Kugelkopf und ist den Hüftgelenk-Endoprothesen in Form und Größe nachgebildet. Der Hüftgelenkspacer wird mit Knochenzement am proximalen Femur verankert. Der Hüftgelenkspacer verbleibt bis zu mehreren Wochen im Patienten bis die Entzündung abgeklungen ist und die klinischen Entzündungsmarker zurückgegangen sind. Dann wird in einer zweiten OP der Hüftgelenkspacer entfernt und nach erneutem Debridement eine Revisions-Hüftgelenk-Endoprothese implantiert.

Die US 2010/0042213 A1 offenbart eine Hüftgelenkprothese mit einem Reservoir einer Flüssigkeit im Inneren des Implantats. Aus der WO 2017/178951 A1 ist ein Hüftspacer mit Vertiefungen bekannt, wobei in den Vertiefungen eine Substanz zur Behandlung des Knochens eingebracht werden kann. In der US 6 245 111 B1 wird eine Hüftgelenksprothese vorgeschlagen, deren Oberflächen mit einem Antibiotikum beschichtet sind. Das Patent US 5 681 289 B1 offenbart eine Einrichtung zum Verteilen eines flüssigen Wirkstoffs mit Hilfe einer Blase im Inneren der Einrichtung.

Es ist bekannt, mit Antibiotika ausgerüstete Spacer zu verwenden. Hüftgelenkspacer können einerseits vom OP-Personal während der OP selbst aus PMMA-Knochenzementpulver, Antibiotika und Monomerflüssigkeit hergestellt werden, beispielsweise mit einer Spacerform, wie sie beispielsweise in den Patenten DE 10 2015 104 704 B4 oder EP 2 617 393 B1 beschrieben sind, und andererseits ist es auch üblich, industriell aus Knochenzement vorgefertigte Hüftgelenkspacer einzusetzen.

Bei den bisherigen Spacern werden dem Zementpulver vor der eigentlichen Spacerherstellung Antibiotika zugesetzt. Mit diesem antibiotisch modifizierten Knochenzementpulver werden anschließend Spacer gegossen, die dann durch Polymerisation mit Hilfe einer dem Zementpulver zugesetzten Monomerflüssigkeit aushärten. Der Knochenzementteig schließt dabei die Antibiotika im Wesentlichen ein. Nur die in den Oberflächen-nahen Bereichen befindlichen Antibiotika-Partikel werden bei Einwirkung von Körperflüssigkeiten, wie Wundsekret, herausgelöst. Die Wirkstofffreisetzung ist initial am höchsten und nimmt dann im Verlauf von mehreren Tagen ab. Danach werden nur noch geringe Mengen der Antibiotika freigesetzt. Die Hauptmenge der zugesetzten Antibiotika verbleibt im ausgehärteten Knochenzement der Spacer. Eine nachträgliche Veränderung der Art und der Anzahl der eingesetzten Antibiotika ist nach der Spacerherstellung bzw. nach der Implantation bei den bisherigen aus Knochenzement gefertigten Spacern nicht möglich. Weiterhin ist die Einstellung einer definierten Konzentration an antimikrobiellen Wirkstoffen im Wundsekret beziehungsweise der den Spacer umgebenden Körperflüssigkeit ebenfalls nicht möglich.

Die US 2011/0015754 A1 offenbart einen Hüftspacer, der in seinem Inneren ein Flüssigkeitsreservoir enthält, aus dem eine Flüssigkeit abgegeben werden kann. Aus der US 2018/0028320 A1 ist ein Implantat zur Abgabe eines Schmiermittels bekannt.

Im Patent EP 1 991 170 B1 wird ein Hüftgelenkspacer beschrieben, bei dem der Kugelkopf und der Schaft vom Anwender zusammengesteckt werden können, wobei der Kugelkopf einen ersten Wirkstoff enthält und der Schaft mit einem zweiten Wirkstoff ausgerüstet ist.

Ein weiterer Hüftgelenkspacer wird in der US 2011/0015754 A1 offenbart. Bei diesem Spacersystem sind in dem Kugelkopf zwei zylinderförmige Hohlräume vorgesehen, die mit einer Schmalseite auf die Kugeloberfläche münden. Die beiden Hohlräume sind jeweils mit einem flüssigkeitsdurchlässigen Deckel verschlossen. Beide Hohlräume können mit antibiotischen Lösungen befüllt werden. Nach der Implantation migrieren die antibiotischen Lösungen durch die flüssigkeitsdurchlässigen Deckel auf die Oberfläche des Spacers.

Bei der zweizeitigen septischen Revision werden bei der Implantation der Hüftgelenkspacer auch Drainagen eingesetzt, die zur Ableitung von Wundsekret, Blut und Debris bestimmt sind. Die Drainagen verbleiben bis zu mehreren Tagen im Patienten. Die von dem Spacer freigesetzten antibiotischen Wirkstoffe werden vom Wundsekret aufgenommen und über die Drainage nach außen abgeführt. Dadurch geht ein Teil der antimikrobiellen Wirkstoffe zum Schutz der Spaceroberfläche vor mikrobieller Besiedlung verloren.

US2013/211369 A1 beschreibt einen femoralen Hüftgelenkspacer mit einem Schaft mit einer Befestigungsfläche und weiters mit einem Verbindungsmittel zum Zuführen einer medizinischen Spülflüssigkeit in den Prothesenkörper, eine Spülflüssigkeits-Einlassöffnung in einer Oberfläche des Prothesenkörpers, zumindest eine Spülflüssigkeitsaustrittsöffnung, die in der Oberfläche des Prothesenkörpers außerhalb der Befestigungsfläche angeordnet ist, wobei die Spülflüssigkeitsaustrittsöffnung im Inneren des Prothesenkörpers mit der Spülflüssigkeits-Einlassöffnung flüssigkeitsdurchlässig verbunden ist.

Es wurde im Rahmen der vorliegenden Erfindung erkannt, dass es wünschenswert wäre, wenn die Spaceroberfläche von einer antimikrobiellen Wirkstofflösung umgeben wäre, deren Wirkstoffkonzentration exakt eingestellt werden kann und deren Konzentration über mehrere Tage unabhängig vom Wundsekretstrom erhalten bleiben würde. Weiterhin wäre es wünschenswert, dass man die Art und die Anzahl der mikrobiellen Wirkstoffe auch nach der Implantation des Hüftgelenkspacers variieren kann, um zum Beispiel auf erst später nachgewiesene mikrobielle Keime reagieren zu können. Gleichzeitig soll der Patient das Hüftgelenk bewegen können, um eine Verkürzung der Sehnen und der Muskulatur und eine Degeneration der Muskulatur zu verhindern und so die Rehabilitation zu verkürzen.

Die Aufgabe der vorliegenden Erfindung besteht also darin, die Nachteile des Stands der Technik zu überwinden. Insbesondere besteht die Aufgabe der Erfindung in der Entwicklung eines temporären femoralen Hüftgelenkspacers, mit dem eine medizinische Spülflüssigkeit gezielt im Bereich der Hüfte eingesetzt werden kann. Gleichzeitig soll der Hüftgelenkspacer eine Beweglichkeit des Hüftgelenks im beim Patienten eingesetzten Zustand ermöglichen. Die Aufgabe der Erfindung ist es dabei, einen artikulierenden Hüftgelenkspacer zu entwickeln, der für die Interimsphase von zweizeitigen septischen Revisionen von Hüftgelenk-Endoprothesen bestimmt ist und der hierfür nutzbringende Eigenschaften aufweist. Der Hüftgelenkspacer soll den Raum nach der Entfernung der Hüftgelenk-Endoprothese und dem nachfolgenden Debridement so ausfüllen, dass eine Rückbildung des Bandapparates und der Muskulatur verhindert wird. Der zu entwickelnde Hüftgelenkspacer soll es ermöglichen, dass die artikulierende Spaceroberfläche, das den Hüftgelenkspacer umgebende Weichgewebe und zumindest ein Teil des umgebenden Knochengewebes mit antiseptischen oder antibiotischen Spülflüssigkeiten kontinuierlich oder auch diskontinuierlich gespült werden können. Der Hüftgelenkspacer soll mit dem Knochengewebe des proximalen Femurs in der Weise mit Knochenzement verbunden werden können, dass der Austritt der Spülflüssigkeit aus dem Schaft oder im Bereich des Schafts des Hüftgelenkspacers und auch die Aufnahme der Spülflüssigkeit in den Hüftgelenkspacer zur Ableitung nicht gestört oder unterbrochen wird.

Der Hüftgelenkspacer soll ferner möglichst so beschaffen sein, dass nach einer Beendigung der Spülungen mit der Spülflüssigkeit die Spülflüssigkeitszuführung und die Spülflüssigkeitsableitung entfernt werden können, ohne dass die Artikulation des Hüftgelenkspacers beeinträchtigt wird.

Die Aufgaben der Erfindung werden gelöst durch einen femoralen Hüftgelenkspacer zum zeitweisen Ersetzen eines Teils eines Hüftgelenks, der Hüftgelenkspacer aufweisend
A) einen Prothesenkörper, der Prothesenkörper aufweisend einen Kugelkopf mit einer Gleitfläche, einen Hals, der mit dem Kugelkopf verbunden ist, einen Kragen, der an einer proximalen Seite mit dem Hals verbunden ist, und einen Schaft, der mit einer distalen Seite des Kragens verbunden ist, wobei zumindest eine Befestigungsfläche am Schaft des Prothesenkörpers angeordnet ist,
B) der Hüftgelenkspacer ferner aufweisend ein erstes röhrenförmiges und flüssigkeitsdurchlässiges Verbindungsmittel zum Zuführen einer medizinischen Spülflüssigkeit in den Prothesenkörper,
C) ein zweites röhrenförmiges und flüssigkeitsdurchlässiges Verbindungsmittel zum Abführen der Spülflüssigkeit aus dem Prothesenkörper,
D) eine Spülflüssigkeits-Einlassöffnung in einer Oberfläche des Prothesenkörpers, wobei das erste Verbindungsmittel mit der Spülflüssigkeits-Einlassöffnung flüssigkeitsdurchlässig verbunden oder verbindbar ist,
E) eine Spülflüssigkeits-Auslassöffnung in der Oberfläche des Prothesenkörpers, wobei das zweite Verbindungsmittel mit der Spülflüssigkeits-Auslassöffnung flüssigkeitsdurchlässig verbunden oder verbindbar ist,
F) zumindest eine Spülflüssigkeitsaustrittsöffnung und zumindest eine Spülflüssigkeitseintrittsöffnung, die in der Oberfläche des Prothesenkörpers außerhalb der zumindest einen Befestigungsfläche angeordnet sind, wobei
die zumindest eine Spülflüssigkeitsaustrittsöffnung im Inneren des Prothesenkörpers mit der Spülflüssigkeits-Einlassöffnung flüssigkeitsdurchlässig verbunden ist und die zumindest eine Spülflüssigkeitseintrittsöffnung im Inneren des Prothesenkörpers mit der Spülflüssigkeits-Auslassöffnung flüssigkeitsdurchlässig verbunden wobei,
die zumindest eine Spülflüssigkeitsaustrittsöffnung im Inneren des Prothesenkörpers nicht flüssigkeitsdurchlässig mit der Spülflüssigkeits-Auslassöffnung verbunden ist und die zumindest eine Spülflüssigkeitseintrittsöffnung im Inneren des Prothesenkörpers nicht flüssigkeitsdurchlässig mit der Spülflüssigkeits-Einlassöffnung verbunden ist.

Die Spülflüssigkeit kann theoretisch auch zunächst durch das erste Verbindungsmittel eingeleitet werden und durch das zweite Verbindungsmittel abgeführt werden und anschließend durch das zweite Verbindungsmittel eingeleitet werden und durch das erste Verbindungsmittel abgeführt werden. Der Hüftgelenkspacer wird dann alternierend betrieben. Es wird jedoch erfindungsgemäß bevorzugt, dass der Hüftgelenkspacer nur in einer Fließrichtung der Spülflüssigkeit betrieben wird beziehungsweise zu betreiben ist. Vorzugsweise ist das erste röhrenförmige flüssigkeitsdurchlässige Verbindungsmittel ein Schlauch mit einem Adapter oder einem anderen Anschluss.

Vorzugsweise ist das zweite röhrenförmige flüssigkeitsdurchlässige Verbindungsmittel ein Schlauch mit einem Adapter oder einem anderen Anschluss.

In der vorliegenden Patentanmeldung werden die Richtungsbezeichnungen "proximal", "distal" und "lateral" sowie die Ebenen-bezeichnungen "Sagittalebene", "Frontalebene" und "Transversalebene" in Bezug auf den Hüftgelenkspacer so verwendet, wie dies bei dem im Patienten eingesetzten Zustand als anatomische Hauptrichtung oder als Körperebene zu verstehen wäre. Dabei bedeutet "proximal" zum Körperzentrum hin und "distal" vom Körperzentrum entfernt.

Bevorzugt kann vorgesehen sein, dass der Hüftgelenkspacer zur Anwendung eines antibiotischen Wirkstoffs geeignet ist, der eine Polymerisation oder eine radikalische Polymerisation von PMMA verhindert oder beeinträchtigt. Insbesondere kann vorgesehen sein, dass der Hüftgelenkspacer zur Anwendung von Rifampicin und Metronidazol geeignet ist.

Bei erfindungsgemäßen Hüftgelenkspacern kann vorgesehen sein, dass die zumindest eine Befestigungsfläche begrenzt ist oder die zumindest eine Befestigungsfläche mit einem umlaufenden, sich aus der Oberfläche des Prothesenkörpers erhebenden Steg begrenzt ist, so dass die zumindest eine Befestigungsfläche zur Aufnahme von Knochenzementteig innerhalb des Stegs geeignet ist.

Hierdurch kann ein begrenzter und dadurch ein bestimmter Bereich zur Befestigung des Prothesenkörpers verwendet werden. Bei korrekter Anwendung des Hüftgelenkspacers kann dadurch verhindert werden, dass das erste und das zweite Verbindungsmittel sowie die Spülflüssigkeits-Einlassöffnung und die Spülflüssigkeits-Auslassöffnung mit Knochenzement bedeckt werden und dadurch in ihrer Funktion beeinträchtigt werden. Insbesondere kann verhindert werden, dass der ausgehärtete Knochenzement ein Abziehen beziehungsweise ein Lösen des ersten und des zweiten Verbindungsmittels vom Prothesenkörper verhindert.

Dabei kann vorgesehen sein, dass wenigstens eine der zumindest einen Befestigungsfläche bereichsweise durch einen Teil des Kragens begrenzt ist.

Hierdurch wird eine besonders stabile Verbindung des Hüftgelenkspacers mit dem Femur erreicht. Gleichzeitig bleibt aber noch Raum für die zumindest eine Spülflüssigkeitsaustrittsöffnung und für die zumindest eine Spülflüssigkeitseintrittsöffnung außerhalb der zumindest einen Befestigungsfläche, so dass in diesem Bereich mit der Spülflüssigkeit gespült werden kann, ohne dass die Öffnungen mit dem Knochenzementteig zur Fixierung des Hüftgelenkspacers am Femur verschlossen werden.

Gemäß einer besonders bevorzugten Ausführung des erfindungsgemäßen Hüftgelenkspacers kann vorgesehen sein, dass zumindest eine erste Spülflüssigkeitsaustrittsöffnung und zumindest eine erste Spülflüssigkeitseintrittsöffnung in der Oberfläche des Prothesenkörpers an der distalen Seite des Kragens oder am Schaft außerhalb der zumindest einen Befestigungsfläche angeordnet sind, und zumindest eine zweite Spülflüssigkeitsaustrittsöffnung und zumindest eine zweite Spülflüssigkeitseintrittsöffnung in der Oberfläche des Prothesenkörpers seitlich am Kragen oder an der proximalen Seite des Kragens oder am Hals oder am Kugelkopf angeordnet sind, wobei die zumindest eine erste Spülflüssigkeitsaustrittsöffnung und die zumindest eine zweite Spülflüssigkeitsaustrittsöffnung im Inneren des Prothesenkörpers mit der Spülflüssigkeits-Einlassöffnung flüssigkeitsdurchlässig verbunden sind und wobei die zumindest eine erste Spülflüssigkeitseintrittsöffnung und die zumindest eine zweite Spülflüssigkeitseintrittsöffnung im Inneren des Prothesenkörpers mit der Spülflüssigkeits-Auslassöffnung flüssigkeitsdurchlässig verbunden sind.

Hierdurch werden zwei getrennte Kreisläufe der Spülflüssigkeit erzeugte, die getrennt voneinander auf der distalen und der proximalen Seite des Hüftgelenkspacers verlaufen. Der Kragen, der den Fluss der Spülflüssigkeit behindern würde, muss dann nicht umflossen werden, um beide Seiten des Hüftgelenkspacers effektiv mit der Spülflüssigkeit spülen zu können.

Dabei kann vorgesehen sein, dass die zumindest eine erste Spülflüssigkeitsaustrittsöffnung und die zumindest eine erste Spülflüssigkeitseintrittsöffnung voneinander beabstandet sind und die zumindest eine zweite Spülflüssigkeitsaustrittsöffnung und die zumindest eine zweite Spülflüssigkeitseintrittsöffnung voneinander beabstandet sind, wobei der Abstand zumindest 5 mm, bevorzugt zumindest 20 mm und besonders bevorzugt zumindest 30 mm beträgt.

Hierdurch wird sichergestellt, dass der Kreislauf der medizinischen Spülflüssigkeit eine größere Strecke (zumindest 5 mm) an der äußeren Oberfläche des Prothesenkörpers zurücklegen muss und dort zum Spülen zur Verfügung steht.

Bei Hüftgelenkspacern mit Spülflüssigkeitseintrittsöffnungen und Spülflüssigkeitsaustrittsöffnungen zur Herstellung eines proximalen und eines distalen Kreislaufs der Spülflüssigkeit kann vorgesehen sein, dass die zumindest eine erste Spülflüssigkeitsaustrittsöffnung und die zumindest eine erste Spülflüssigkeitseintrittsöffnung durch zumindest einen umlaufenden und sich aus der Oberfläche des Prothesenkörpers erhebenden Steg von der zumindest einen Befestigungsfläche abgegrenzt sind.

Bei korrekter Anwendung der des Hüftgelenkspacers kann dadurch verhindert werden, dass das erste und das zweite Verbindungsmittel sowie die Spülflüssigkeits-Einlassöffnung und die Spülflüssigkeits-Auslassöffnung sowie die zumindest eine erste Spülflüssigkeitsaustrittsöffnung und die zumindest eine erste Spülflüssigkeitseintrittsöffnung mit Knochenzement bedeckt werden und dadurch in ihrer Funktion beeinträchtigt werden. Insbesondere kann verhindert werden, dass der ausgehärtete Knochenzement ein Abziehen beziehungsweise ein Lösen des ersten und des zweiten Verbindungsmittels vom Prothesenkörper verhindert.

Ferner kann vorgesehen sein, dass wenigstens eine Spülflüssigkeitsaustrittsöffnung der zumindest einen zweiten Spülflüssigkeitsaustrittsöffnung und wenigstens eine Spülflüssigkeitseintrittsöffnung der zumindest einen zweiten Spülflüssigkeitseintrittsöffnung neben der Gleitfläche am Kugelkopf angeordnet sind, bevorzugt innerhalb von 5 mm neben der Gleitfläche am Kugelkopf angeordnet sind.

Hiermit wird sichergestellt, dass die zumindest eine zweite Spülflüssigkeitsaustrittsöffnung und die zumindest eine zweite Spülflüssigkeitseintrittsöffnung nicht die Funktion der Gleitfläche beeinträchtigen. Zudem kann so eine mechanische Belastung und ein damit einhergehender unerwünschter Abrieb der Kanten der zumindest einen zweiten Spülflüssigkeitsaustrittsöffnung und der zumindest einen zweiten Spülflüssigkeitseintrittsöffnung verhindert werden.

Des Weiteren kann vorgesehen sein, dass wenigstens eine der zumindest einen ersten Spülflüssigkeitsaustrittsöffnung am Ende des Schafts angeordnet ist und wenigstens eine der zumindest einen ersten Spülflüssigkeitseintrittsöffnung an der distalen Seite des, am Übergang vom Kragen zum Schaft oder an der proximalen Seite des Schafts angeordnet ist.

Hierdurch wird erreicht, dass die Spülflüssigkeit die gesamte Länge des Schafts auf der distalen Seite des Prothesenkörpers mit der medizinischen Spülflüssigkeit spülen kann, so dass auch ein Durchspülen des Femurs bis in die erreichbare Tiefe erfolgen kann.

Des Weiteren kann vorgesehen sein, dass die zumindest eine Spülflüssigkeitsaustrittsöffnung im Inneren des Prothesenkörpers nicht flüssigkeitsdurchlässig mit der Spülflüssigkeits-Auslassöffnung verbunden ist und dass die zumindest eine Spülflüssigkeitseintrittsöffnung im Inneren des Prothesenkörpers nicht flüssigkeitsdurchlässig mit der Spülflüssigkeits-Einlassöffnung verbunden ist.

Hierdurch wird sichergestellt, dass mit dem Hüftgelenkspacer ein Kreislauf mit der Spülflüssigkeit erzeugt werden kann, ohne dass die Spülflüssigkeit zunächst eingedrückt und anschließend abgesaugt werden muss. Der Hüftgelenkspacer kann alternativ aber auch so verwendet werden, dass die Spülflüssigkeit zunächst in den Hüftgelenkspacer eingeleitet wird und durch die zumindest eine Spülflüssigkeitsaustrittsöffnungen (bevorzugt durch die zumindest eine erste Spülflüssigkeitsaustrittsöffnung und die zumindest eine zweite Spülflüssigkeitsaustrittsöffnung) und durch die zumindest eine Spülflüssigkeitseintrittsöffnung (bevorzugt durch die zumindest eine erste Spülflüssigkeitseintrittsöffnung und die zumindest eine zweite Spülflüssigkeitseintrittsöffnung) austritt und anschließend die Spülflüssigkeit wieder abgesaugt wird und dabei durch die zumindest eine Spülflüssigkeitsaustrittsöffnung und die zumindest eine Spülflüssigkeitseintrittsöffnung wieder in den Prothesenkörper eingesaugt wird.

Ferner kann vorgesehen sein, dass die zumindest eine Spülflüssigkeitsaustrittsöffnung und die zumindest eine Spülflüssigkeitseintrittsöffnung voneinander beabstandet sind, wobei der Abstand zumindest 5 mm, bevorzugt zumindest 20 mm und besonders bevorzugt zumindest 30 mm beträgt.

Hierdurch wird sichergestellt, dass der Kreislauf der medizinischen Spülflüssigkeit eine größere Strecke (zumindest 5 mm) an der äußeren Oberfläche des Prothesenkörpers zurücklegen muss und dort zum Spülen zur Verfügung steht.

Gemäß einer bevorzugt Weiterbildung des erfindungsgemäßen Hüftgelenkspacers kann vorgesehen sein, dass das erste Verbindungsmittel an der zur Verbindung mit der Spülflüssigkeits-Einlassöffnung abgewandten Seite und das zweite Verbindungsmittel an der zur Verbindung mit der Spülflüssigkeits-Auslassöffnung abgewandten Seite jeweils einen Adapter aufweist, insbesondere jeweils einen Luer-Lock-Adapter aufweist.

Hierdurch kann der Hüftgelenkspacer bequem an ein medizinisches Spülflüssigkeitsreservoir mit einer Pumpe und an einen Auffangbehälter zum Aufnahmen der gebrauchten Spülflüssigkeit angeschlossen werden.

Es kann auch vorgesehen sein, dass an der Spülflüssigkeits-Einlassöffnung im Inneren des Prothesenkörpers oder an der Oberfläche des Prothesenkörpers eine selbstdichtende Kupplung angeordnet ist und an der Spülflüssigkeits-Auslassöffnung im Inneren des Prothesenkörpers oder an der Oberfläche des Prothesenkörpers eine selbstdichtende Kupplung angeordnet ist, wobei das erste Verbindungsmittel lösbar mit der Spülflüssigkeits-Einlassöffnung verbunden oder verbindbar ist und das zweite Verbindungsmittel lösbar mit der Spülflüssigkeits-Auslassöffnung verbunden oder verbindbar ist.

Hierdurch verschließen sich die Spülflüssigkeits-Einlassöffnung und die Spülflüssigkeits-Auslassöffnung beziehungsweise die Flüssigkeitsleitungen dahinter selbsttätig, wenn das erste Verbindungsmittel oder das zweite Verbindungsmittel vom Prothesenkörper abgezogen beziehungsweise getrennt wird. Dadurch wird der Durchgang verschlossen, wenn keine Spülflüssigkeit mehr durch den Hüftgelenkspacer geleitet werden soll.

Ferner kann vorgesehen sein, dass die Spülflüssigkeits-Einlassöffnung eine erste Spülflüssigkeits-Einlassöffnung ist und die Spülflüssigkeits-Auslassöffnung eine erste Spülflüssigkeits-Auslassöffnung ist, wobei zusätzlich eine zweite Spülflüssigkeits-Einlassöffnung und eine zweite Spülflüssigkeits-Auslassöffnung in der Oberfläche des Prothesenkörpers vorgesehen sind, wobei an der ersten Spülflüssigkeits-Einlassöffnung, der zweiten Spülflüssigkeits-Einlassöffnung, der ersten Spülflüssigkeits-Auslassöffnung und der zweiten Spülflüssigkeits-Auslassöffnung jeweils eine selbstdichtende Kupplung angeordnet ist, wobei das erste Verbindungsmittel flüssigkeitsdicht und lösbar mit der ersten Spülflüssigkeits-Einlassöffnung und mit der zweiten Spülflüssigkeits-Einlassöffnung verbindbar ist und das zweite Verbindungsmittel flüssigkeitsdicht und lösbar mit der ersten Spülflüssigkeits-Auslassöffnung und mit der zweiten Spülflüssigkeits-Auslassöffnung verbindbar ist und wobei die erste Spülflüssigkeits-Einlassöffnung und die zweite Spülflüssigkeits-Einlassöffnung im Prothesenkörper miteinander flüssigkeitsdurchlässig verbunden sind und die erste Spülflüssigkeits-Auslassöffnung und die zweite Spülflüssigkeits-Auslassöffnung im Prothesenkörper miteinander flüssigkeitsdurchlässig verbunden sind.

Hierdurch kann der Zugang zum Hüftgelenkspacer variabel gewählt werden und an die jeweilige Situation individuell und patientenspezifisch angepasst werden.

Bevorzugt kann auch vorgesehen sein, dass die Summe der Querschnittsflächen aller der zumindest einen Spülflüssigkeitseintrittsöffnung zusammen zumindest genauso groß ist wie die Querschnittsfläche der Spülflüssigkeits-Einlassöffnung und/oder die Summe aller der Querschnittsflächen der zumindest einen Spülflüssigkeitsaustrittsöffnung zumindest genauso groß ist wie die Querschnittsfläche der Spülflüssigkeits-Auslassöffnung.

Hierdurch kann ein Staudruck im Inneren des Prothesenkörpers vermieden werden.

Bevorzugt kann ferner vorgesehen sein, dass die zumindest eine Spülflüssigkeitsaustrittsöffnung und die zumindest eine Spülflüssigkeitseintrittsöffnung nicht in der Gleitfläche des Kugelkopfs angeordnet sind.

Hiermit wird sichergestellt, dass die zumindest eine Spülflüssigkeitsaustrittsöffnung und die zumindest eine Spülflüssigkeitseintrittsöffnung nicht die Funktion der Gleitfläche beeinträchtigen. Zudem kann so eine mechanische Belastung und ein damit einhergehender unerwünschter Abrieb der Kanten der zumindest einen Spülflüssigkeitsaustrittsöffnung und der zumindest einen Spülflüssigkeitseintrittsöffnung verhindert werden.

Des Weiteren kann vorgesehen sein, dass in dem ersten Verbindungsmittel oder in der Spülflüssigkeits-Einlassöffnung ein erstes Ventilelement angeordnet ist, das ein Rückfließen der Spülflüssigkeit in das erste Verbindungsmittel verhindert, und/oder in dem zweiten Verbindungsmittel oder in der Spülflüssigkeits-Auslassöffnung ein zweites Ventilelement angeordnet ist, das ein Rückfließen der Spülflüssigkeit in das zweite Verbindungsmittel verhindert, wobei bevorzugt das erste und/oder das zweite Ventilelement ausgewählt sind aus einem Rückschlagventil, einem Kugelventil mit Feder, einem Lippenventil, einem Bunsenventil oder einem Plattenventil.

Dadurch kann ein umlaufender Kreislauf der medizinischen Spülflüssigkeit vorgegeben werden. Zudem kann so ein Rücklaufen der benutzten medizinischen Spülflüssigkeit verhindert werden.

Mit einer bevorzugten Weiterbildung der vorliegenden Erfindung wird vorgeschlagen, dass in einer ersten Leitung innerhalb des Prothesenkörpers, die die zumindest eine Spülflüssigkeitseintrittsöffnung mit der Spülflüssigkeits-Auslassöffnung flüssigkeitsdurchlässig verbindet, ein erstes Ventil angeordnet ist, das nur durch Anlegen eines Unterdrucks an der Spülflüssigkeits-Auslassöffnung zu öffnen ist und das ein Zurückfließen der Spülflüssigkeit in die erste Leitung verhindert, und/oder in einer zweiten Leitung innerhalb des Prothesenkörpers, die die zumindest eine Spülflüssigkeitsaustrittsöffnung mit der Spülflüssigkeits-Einlassöffnung flüssigkeitsdurchlässig verbindet, ein zweites Ventil angeordnet ist, das nur durch Anlegen eines Unterdrucks an der Spülflüssigkeits-Einlassöffnung zu öffnen ist und das ein Zurückfließen der Spülflüssigkeit in die zweite Leitung verhindert.

Auch dadurch kann ein Rücklaufen der medizinischen Spülflüssigkeit verhindert werden. Zudem kann so sichergestellt werden, dass ohne die Verbindungsmittel noch ein Austausch enthaltener Spülflüssigkeit mit umgebenden Flüssigkeiten stattfindet.

Es kann auch vorgesehen sein, dass in dem Prothesenkörper die Spülflüssigkeits-Einlassöffnung, die Spülflüssigkeits-Auslassöffnung, die zumindest eine Spülflüssigkeitsaustrittsöffnung und die zumindest eine Spülflüssigkeitseintrittsöffnung sowie die flüssigkeitsdurchlässigen Verbindungen ausgebildet sind, wobei der Prothesenkörper vorzugsweise aus Kunststoff, Metall, Keramik, Glaskeramik, Knochenzement oder einer Kombination daraus gefertigt ist.

Hierdurch wird ein kompakter Aufbau erreicht und der Prothesenkörper gleicht äußerlich bis auf die Öffnungen einem herkömmlichen femoralen Hüftgelenkspacer.

Des Weiteren kann vorgesehen sein, dass die Spülflüssigkeits-Einlassöffnung und die Spülflüssigkeits-Auslassöffnung in einer lateralen Oberfläche des Halses, der distalen Seite des Kugelkopfs oder des Kragens, insbesondere der proximalen Seite des Kragens, angeordnet sind.

Hierdurch kann Zufuhr und Abfuhr der Spülflüssigkeit in den und aus dem Hüftgelenkspacer anatomisch leicht und bequem gelegt werden. An der lateralen seitlichen Oberfläche stören die an die Spülflüssigkeits-Einlassöffnung und die Spülflüssigkeits-Auslassöffnung angeschlossenen Verbindungsmittel beim Laufen besonders wenig und sind auch besonders leicht zugänglich.

Bevorzugt kann ferner vorgesehen sein, dass wenigstens eine Spülflüssigkeitsaustrittsöffnung der zumindest einen Spülflüssigkeitsaustrittsöffnung am Ende des Schafts angeordnet ist.

Hierdurch wird erreicht, dass die Spülflüssigkeit auch am Ende des Schafts mit der medizinischen Spülflüssigkeit spülen kann, so dass auch ein Durchspülen des Femurs bis in die erreichbare Tiefe erfolgen kann.

Des Weiteren kann vorgesehen sein, dass der Prothesenkörper mehrteilig ist und einen Schaftteil und wenigstens einen Kopfteil aufweist, die über ein Verbindungsmittel, insbesondere über eine Schraubverbindung, miteinander lösbar verbindbar sind oder lösbar verbunden sind, vorzugsweise der Prothesenkörper aufweisend einen Schaftteil und mehrere Kopfteile, wobei die Kopfteile Kugelköpfe mit unterschiedlichen Durchmessern haben.

Hierdurch wird ein variabler und an unterschiedliche Behandlungssituationen anpassbarer Hüftgelenkspacer bereitgestellt.

Der Erfindung liegt die überraschende Erkenntnis zugrunde, dass ein temporärer Hüftgelenkspacer zum kontinuierlichen Spülen einer Kavität im Körper eines Patienten verwendet werden kann, indem an der Oberfläche des Prothesenkörpers des Hüftgelenkspacers geeignete Öffnungen und im Inneren des Prothesenkörpers geeignete Leitungen für die Spülflüssigkeit vorhanden sind und indem zwei von außen zugängliche Verbindungsmittel angeschlossen sind oder anschließbar sind, durch die die medizinische Spülflüssigkeit von außen in den Prothesenkörper eingespeist und die verbrauchte Spülflüssigkeit wieder aus dem Prothesenkörper abgeführt werden kann. Bei dem Hüftgelenkspacer können bei einer bevorzugten Ausführung sowohl eine distale als auch eine proximale Seite durch getrennte Kreisläufe der Spülflüssigkeit gespült werden, so dass der Kragen des Hüftgelenkspacers, der zur Auflage am Femur verwendet wird, nicht von der Spülflüssigkeit umflossen werden muss.

Der erfindungsgemäße Hüftgelenkspacer kann vorteilhaft im Rahmen von zweizeitigen septischen Revisionen verwendet werden, bei denen eine Infektion mit zwei oder mehreren mikrobiellen Keimen und insbesondere mit problematischen Keimen vorliegt. Besonders vorteilhaft ist es, dass der Hüftgelenkspacer und das umgebende Weichgewebe und zumindest teilweise auch das umgebende Knochengewebe mit antibiotisch wirksamen Lösungen, wie Antibiotika und auch Antiseptika oder in speziellen Fällen mit Antimykotika, gespült werden können, wobei die Art und die Anzahl der Wirkstoffe und vor allem die Konzentration der antimikrobiellen Wirkstoffe in der Spüllösung (der medizinischen Spülflüssigkeit) exakt eingestellt werden können. Durch Absaugen der Spülflüssigkeit kann auch die Verweildauer der antimikrobiell ausgerüsteten Spülflüssigkeit im Patienten exakt eingestellt werden. Dadurch ist es möglich, über mehrere Tage eine Umspülung der Oberfläche des Hüftgelenkspacers mit exakt zuvor eingestellte Konzentrationen an antimikrobiellen Wirkstoffen in der Spülflüssigkeit zu gewährleisten. Dadurch wird der Schutz vor einer mikrobiellen Wiederbesiedlung der Oberflächen des Hüftgelenkspacers deutlich verringert im Vergleich zu den bisherigen aus antibiotikahaltigem Knochenzement gefertigten Hüftgelenkspacern. Nach der antibiotischen Spülung ist es möglich, die Oberfläche des Hüftgelenkspacers und des umgebenden Gewebes mit Wirkstoff-freien Spülflüssigkeiten zu spülen. Dadurch werden Reste der antimikrobiellen Wirkstoffe entfernt. Eine Resistenzbildung infolge von persistierenden Wirkstoffrückständen ist daher extrem unwahrscheinlich.

Weiterhin ist es vorteilhaft, dass die Spülflüssigkeiten auch solche antimikrobiellen Wirkstoffe enthalten können, die normalerweise nicht in die aus Knochenzement gefertigten Hüftgelenkspacern integriert werden können, weil diese die radikalische Polymerisation des Knochenzementteigs stören beziehungsweise verhindern würden. Exemplarisch dafür seien die Wirkstoffe Rifampicin und Metronidazol genannt.

Wenn die klinischen Parameter erkennen lassen, dass die Infektion beziehungsweise die Entzündung abklingt, dann können die Verbindungsmittel vom Hüftgelenkspacer entfernt werden. Die Verbindungsmittel sind hierzu vorteilhaft mit einem Außengewinde oder über einen Bajonettverschluss oder über einen Steckverschluss mit der Spülflüssigkeits-Einlassöffnung und der Spülflüssigkeits-Auslassöffnung verbunden, beziehungsweise mit jeweils einem zu dem Befestigungselement am Verbindungsmittel passenden Gegenbefestigungselement in oder an der Spülflüssigkeits-Einlassöffnung und der Spülflüssigkeits-Auslassöffn ung.

Bevorzugt kann vorgesehen sein, dass die Spülflüssigkeits-Einlassöffnung und die Spülflüssigkeits-Auslassöffnung nach Entfernung der Verbindungsmittel bündig mit der Oberfläche des Hüftgelenkspacers abschließen, um eine Irritation des umgebenden Weichgewebes zu verhindern.

Ein beispielhafter erfindungsgemäßer Hüftgelenkspacer kann zusammengesetzt sein aus
A) einem Kugelkopf mit einer Gleitfläche,
B) einem Hals der mit dem Kugelkopf verbunden ist,
C) einem Kragen, dessen proximale Seite mit dem Hals verbunden ist,
D) einem Schaft, der mit der distalen Seite des Kragens verbunden ist,
E) mindestens einer Spülflüssigkeits-Einlassöffnung oberhalb der distalen Seite des Kragens, die lösbar mit einem ersten Verbindungsmittel flüssigkeitsdurchlässig verbunden ist,
F) mindestens einer Spülflüssigkeits-Auslassöffnung auf oder oberhalb der proximalen Seite des Kragens, die mit der Spülflüssigkeits-Einlassöffnung flüssigkeitsdurchlässig verbunden ist,
G) mindestens einer Spülflüssigkeits-Auslassöffnung, die auf oder unterhalb der distalen Seite des Kragens angeordnet ist, wobei die Spülflüssigkeits-Auslassöffnung flüssigkeitsdurchlässig mit der Spülflüssigkeits-Einlassöffnung verbunden ist,
H) mindestens einer Spülflüssigkeits-Aufnahmeöffnung, die auf oder oberhalb der distalen Seite des Kragens angeordnet ist, wobei die Spülflüssigkeitsaufnahmeöffnung mit einem zweiten Verbindungsmittel flüssigkeitsdurchlässig verbunden ist,
I) mindestens einer Spülflüssigkeits-Auslassöffnung, die auf oder oberhalb der proximalen Seite des Kragens angeordnet ist, wobei die mindestens eine Spülflüssigkeits-Auslassöffnung mit der Spülflüssigkeits-Aufnahmeöffnung flüssigkeitsdurchlässig verbunden ist,
J) mindestens einer Spülflüssigkeits-Auslassöffnung, die auf oder unterhalb der distalen Seite des Kragens angeordnet ist, wobei diese mindestens eine Spülflüssigkeits-Auslassöffnung mit der Spülflüssigkeits-Aufnahmeöffnung flüssigkeitsdurchlässig verbunden ist, und
K) mindestens einer für die Zementierung vorgesehene Befestigungsfläche am proximalen Schaft, die durch einen umlaufenden Steg abgegrenzt ist.

Vorzugsweise hat die für die Zementierung vorgesehene Befestigungsfläche maximal einen Umfang kleiner dem Außendurchmesser des Schafts.

Der Abstand zwischen den zueinander gehörenden Spülflüssigkeitsaustrittsöffnungen und den Spülflüssigkeitseintrittsöffnungen beträgt mindestens 0,5 cm, bevorzugt mindestens 2,0 cm und ganz bevorzugt mindestens 3,0 cm. Dadurch wird die Spülung der Oberfläche des Hüftgelenkspacers durch die Spülflüssigkeit ermöglicht, bevor die Spülflüssigkeit durch die Spülflüssigkeitseintrittsöffnungen wieder abgesaugt wird.

Es kann erfindungsgemäß auch vorgesehen sein, dass die Spülflüssigkeits-Einlassöffnung und die Spülflüssigkeits-Auslassöffnung im Hals oder im Kragen neben Gleitfläche des Kugelkopfs angeordnet sind. Dadurch können die Verbindungsmittel auf kürzestem Wege vom Hüftgelenkspacer nach außen durch die Körperoberfläche geführt werden.

Die Summe der Querschnitte der Spülflüssigkeitseintrittsöffnungen ist vorzugsweise größer oder gleich dem Querschnitt der Spülflüssigkeitsaustrittsöffnungen.

Es kann erfindungsgemäß auch vorgesehen sein, dass zwischen der zumindest einen Spülflüssigkeitseintrittsöffnung und der Spülflüssigkeits-Auslassöffnung ein Rückschlagventil angeordnet ist, das ein Zurückfließen der Spülflüssigkeit aus dem zweiten Verbindungsmittel in den Prothesenkörper verhindert, wobei als Rückschlagventil ein Kugelventil mit Feder, ein Lippenventil, Bunsenventil und ein Plattenventil bevorzugt sind.

Vorteilhaft ist in einer Leitung im Inneren des Prothesenkörpers ein Rückschlagventil angeordnet, das ein Zurückfließen der Spülflüssigkeit aus der Spülflüssigkeits-Einlassöffnung in das erste Verbindungsmittel verhindert, wobei als Rückschlagventil ein Kugelventil mit Feder, ein Lippenventil, Bunsenventil und ein Plattenventil bevorzugt sind.

Es kann vorgesehen sein, dass in einer Leitung im Inneren des Prothesenkörpers ein Saugventil angeordnet, das ein Zurückfließen der Spülflüssigkeit in die mindestens eine Spülflüssigkeits-Auslassöffnung verhindert, wobei als Saugventil ein Kugelventil mit Feder, ein Lippenventil, Bunsenventil und ein Plattenventil bevorzugt sind.

Der Hüftgelenkspacer kann aus Kunststoff, Metall, Keramik, Glaskeramik und deren Kombinationen gefertigt sein. Vorteilhaft lässt sich der Spacer aus einem Metallkern, der mittels SLM (Selective Laser Melting) hergestellt wurde und einem darum angeordneten Mantel aus Knochenzement fertigen. Es ist auch möglich den gesamten Hüftgelenkspacer aus Metall, wie zum Beispiel Edelstahl und Titanlegierungen, mit SLM zu fertigen. Bevorzugt sind dabei der Edelstahl 1.4404 und die Titanlegierung Ti₆Al₄V.

Im Folgenden werden weitere Ausführungsbeispiele der Erfindung anhand von zweiundzwanzig schematisch dargestellten Figuren erläutert, ohne jedoch dabei die Erfindung zu beschränken. Dabei zeigt:
Figur 1: eine schematische perspektivische Außenansicht eines beispielhaften ersten erfindungsgemäßen Hüftgelenkspacers mit Spülvorrichtung;
Figur 2: eine schematische perspektivische Außenansicht des ersten erfindungsgemäßen Hüftgelenkspacers nach Figur 1 auf die gegenüberliegende Seite;
Figur 3: eine schematische perspektivische Außenansicht des ersten erfindungsgemäßen Hüftgelenkspacers nach Figur 1 und 2 auf eine weitere Seite;
Figur 4: eine schematische Querschnittansicht des ersten erfindungsgemäßen Hüftgelenkspacers entsprechend dem Schnitt A in Figur 3;
Figur 5: eine schematische Querschnittansicht durch einen Ausschnitt des ersten erfindungsgemäßen Hüftgelenkspacers, wobei die Schnittebene parallel zu dem Schnitt A nach Figur 3 verläuft;
Figur 6: eine schematische Seitenansicht des ersten erfindungsgemäßen Hüftgelenkspacers nach den Figuren 1 bis 5, wobei die Blickrichtung der Querschnittansicht nach Figur 4 entspricht;
Figur 7: eine schematische Querschnittansicht des ersten erfindungsgemäßen Hüftgelenkspacers entsprechend dem Schnitt B in Figur 6, wobei die Schnittebene senkrecht zu den Schnittebenen der Figuren 4 und 5 verläuft;
Figur 8: eine schematische perspektivische Außenansicht eines zweiten erfindungsgemäßen Hüftgelenkspacers mit Spülvorrichtung;
Figur 9: eine schematische Seitenansicht des zweiten erfindungsgemäßen Hüftgelenkspacers nach Figur 8;
Figur 10: eine schematische Querschnittansicht des zweiten erfindungsgemäßen Hüftgelenkspacers entsprechend dem Schnitt C in Figur 9;
Figur 11: eine schematische Seitenansicht des zweiten erfindungsgemäßen Hüftgelenkspacers nach den Figuren 8 bis 10 auf die zu Figur 9 gegenüberliegende Seite;
Figur 12: eine schematische Querschnittansicht des zweiten erfindungsgemäßen Hüftgelenkspacers entsprechend dem Schnitt D in Figur 11, wobei die Schnittebene senkrecht zu der Schnittebene der Figur 10 verläuft;
Figur 13: eine schematische perspektivische Außenansicht eines dritten erfindungsgemäßen Hüftgelenkspacers mit Spülvorrichtung;
Figur 14: eine schematische perspektivische Außenansicht des dritten erfindungsgemäßen Hüftgelenkspacers nach Figur 13 auf die gegenüberliegende Seite;
Figur 15: eine schematische Querschnittansicht des dritten erfindungsgemäßen Hüftgelenkspacers nach den Figuren 13 und 14, wobei der Schnitt in der Frontalebene verläuft;
Figur 16: eine schematische Querschnittansicht durch einen Ausschnitt des dritten erfindungsgemäßen Hüftgelenkspacers, wobei die Schnittebene parallel zu der nach Figur 15 verläuft;
Figur 17: eine schematische Querschnittansicht des dritten erfindungsgemäßen Hüftgelenkspacers nach den Figuren 13 bis 16, wobei die Schnittebene senkrecht zu der Schnittebene der Figuren 15 und 16 verläuft;
Figur 18: eine weitere schematische perspektivische Außenansicht des dritten erfindungsgemäßen Hüftgelenkspacers nach den Figuren 13 bis 17 auf das distale Ende des Schafts;
Figur 19: eine schematische Querschnittansicht durch eine Variante des ersten, zweiten und dritten erfindungsgemäßen Hüftgelenkspacers der bis auf die Anordnung von Ventilelementen genauso wie der erste, zweite oder dritte erfindungsgemäße Hüftgelenkspacer aufgebaut ist;
Figur 20: eine schematische perspektivische Außenansicht eines mehrteiligen vierten erfindungsgemäßen Hüftgelenkspacers mit Spülvorrichtung;
Figur 21: eine weitere schematische perspektivische Außenansicht des mehrteiligen vierten erfindungsgemäßen Hüftgelenkspacers nach Figur 20 im auseinandergebauten Zustand; und
Figur 22: eine schematische Querschnittansicht durch den vierten erfindungsgemäßen Hüftgelenkspacer nach den Figuren 20 und 21.

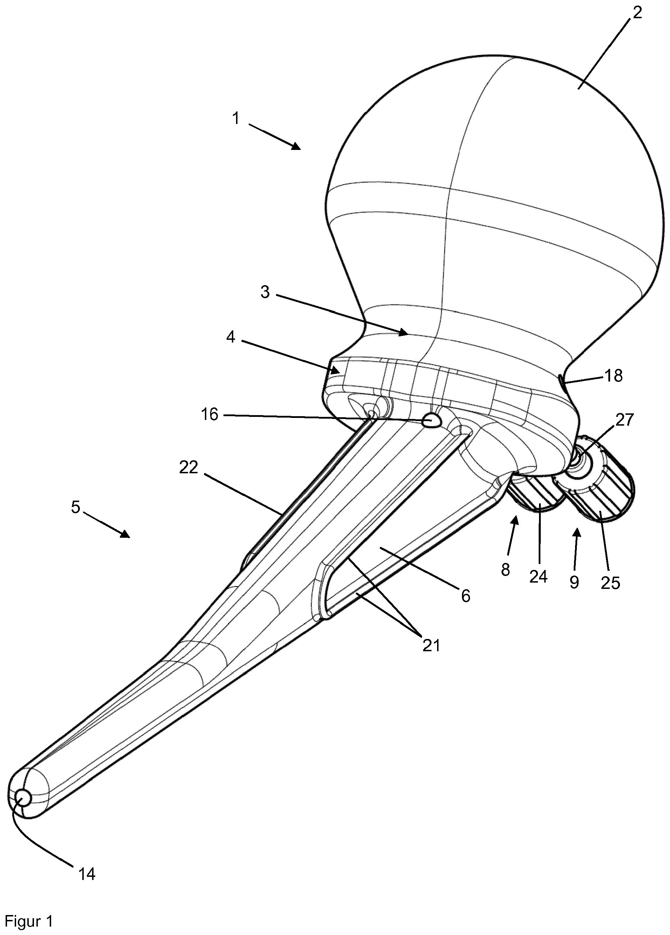

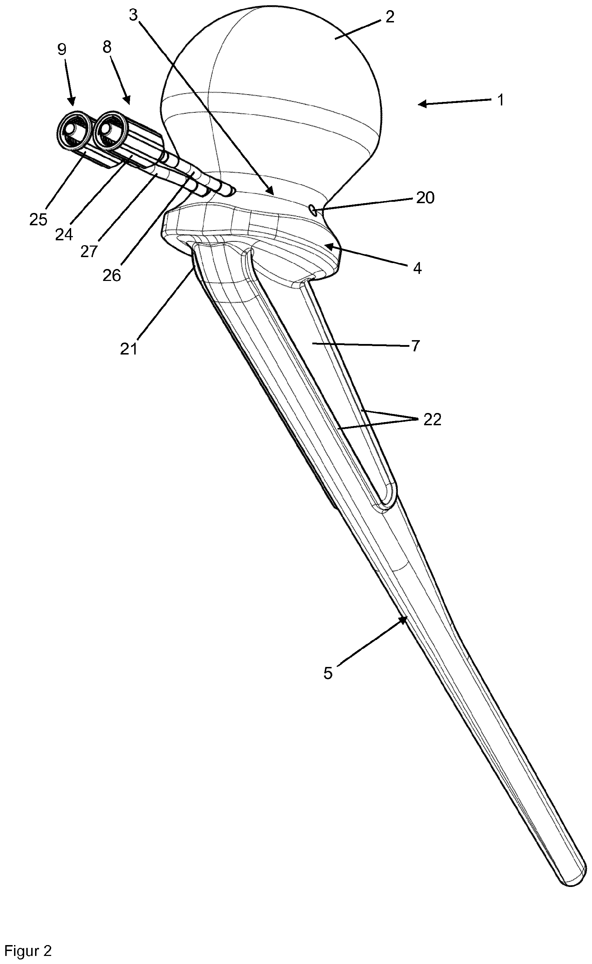

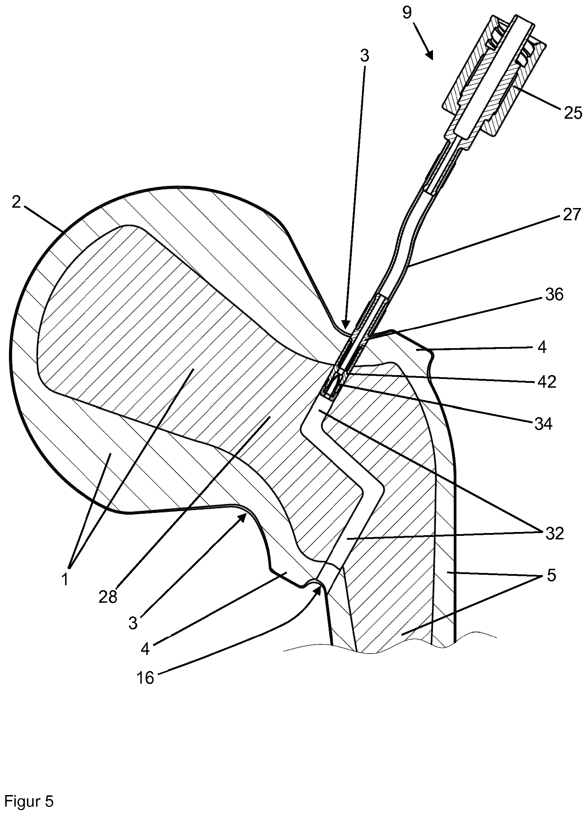

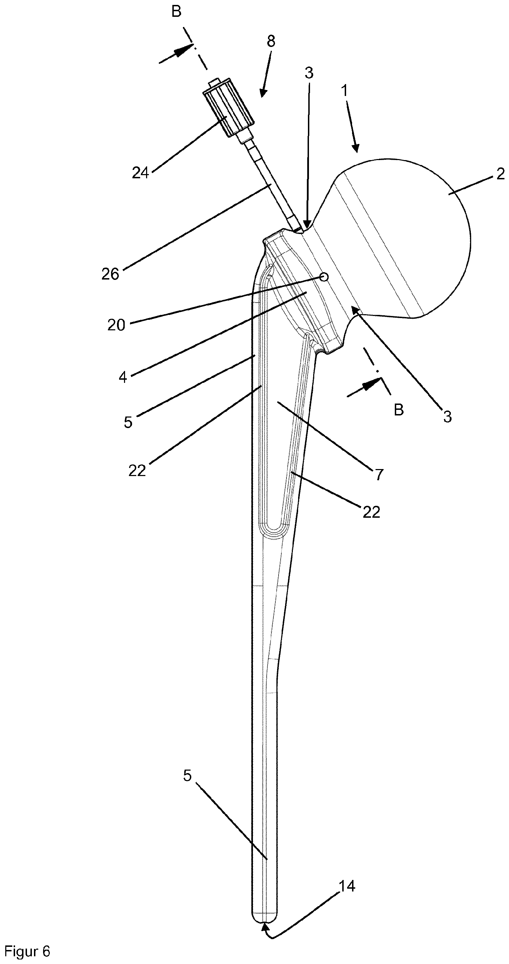

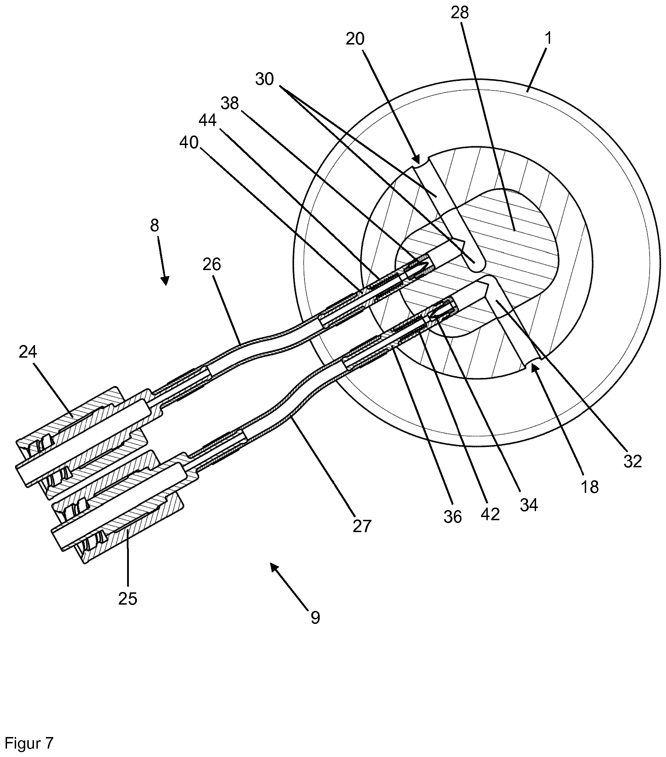

In den Figuren 1 bis 7 sind Abbildungen eines ersten Ausführungsbeispiels eines erfindungsgemäßen Hüftgelenkspacers mit Spülvorrichtung gezeigt. Der femorale Hüftgelenkspacer, das heißt der den Gelenkkopf des Femurs nachbildende und am Femur zu befestigende Hüftgelenkspacer, weist einen Kugelkopf 1 mit einer Gleitfläche 2 an der proximalen Seite auf. Die Gleitfläche 2 liegt im eingesetzten Zustand (d.h. im beim Patienten eingesetzten Zustand) an der Hüftgelenkspfanne an und bildet so einen Teil des Hüftgelenks. Der Kugelkopf 1 ist auf der der Gleitfläche 2 gegenüberliegenden distalen Seite über einen Hals 3 mit einem Kragen 4 verbunden. Der Hals 3 ist dünner als der Kugelkopf 1 und der Kragen 4. An der distalen Seite des Kragens 4 ist ein Schaft 5 befestigt, der sich in die distale Richtung erstreckt und der zur Befestigung des Hüftgelenkspacers im Femur dient. Hierzu weist der Hüftgelenkspacer an zwei gegenüberliegenden Seiten des Schafts 5 Befestigungsflächen 6, 7 auf, die zur Verbindung des Hüftgelenkspacers mit dem Femur mit Hilfe von Knochenzementteig vorgesehen sind. Der Kugelkopf 1, der Hals 3, der Kragen 4 und der Schaft 5 bilden einen Prothesenkörper des Hüftgelenkspacers. Der Prothesenkörper entspricht in seiner äußeren Form weitgehend der äußeren Form bekannter Hüftgelenkspacer.

An einer Seite des ersten beispielhaften Hüftgelenkspacers sind im Unterschied zu bekannten Hüftgelenkspacern ein erstes röhrenförmiges Verbindungsmittel 8 an einer Spülflüssigkeits-Einlassöffnung befestigt und ein zweites röhrenförmiges Verbindungsmittel 9 an einer Spülflüssigkeits-Auslassöffnung befestigt. Die Spülflüssigkeits-Einlassöffnung und die Spülflüssigkeits-Auslassöffnung führen ins Innere des Prothesenkörpers und sind im Bereich des Halses 3 angeordnet. Das erste röhrenförmige Verbindungsmittel 8 und das zweite röhrenförmige Verbindungsmittel 9 sind flüssigkeitsdurchlässig, so dass eine medizinische Spülflüssigkeit durch das erste röhrenförmige Verbindungsmittel 8 in den Prothesenkörper hineingeleitet werden kann und eine Flüssigkeit durch das zweite röhrenförmige Verbindungsmittel 9 aus dem Prothesenkörper abgeführt werden kann. Das erste Verbindungsmittel 8 und das zweite Verbindungsmittel 9 sind lösbar mit der Spülflüssigkeits-Einlassöffnung und der Spülflüssigkeits-Auslassöffnung verbunden.

Am distalen Ende des Schafts 5 ist eine erste Spülflüssigkeitsaustrittsöffnung 14 angeordnet und am Übergang vom Schaft 5 zum Kragen 4 ist eine erste Spülflüssigkeitseintrittsöffnung 16 angeordnet. Des Weiteren ist am Hals 3 beziehungsweise am Übergang vom Hals 3 zum Kragen 4 eine zweite Spülflüssigkeitseintrittsöffnung 18 und auf der zur zweiten Spülflüssigkeitseintrittsöffnung 18 gegenüberliegenden Seite am Hals 3 beziehungsweise am Übergang vom Hals 3 zum Kragen 4 eine zweite Spülflüssigkeitsaustrittsöffnung 20 angeordnet. Die erste Spülflüssigkeitsaustrittsöffnung 14 und die erste Spülflüssigkeitseintrittsöffnung 16 sind dadurch auf der distalen Seite des Hüftgelenkspacers angeordnet und die zweite Spülflüssigkeitsaustrittsöffnung 20 und die zweite Spülflüssigkeitseintrittsöffnung 18 sind auf der proximalen Seite des Hüftgelenkspacers angeordnet, wobei die proximale und die distale Seite durch den Kragen 4 getrennt sind. Die erste Spülflüssigkeitsaustrittsöffnung 14 und die erste Spülflüssigkeitseintrittsöffnung 16 einerseits und die zweite Spülflüssigkeitsaustrittsöffnung 20 und die zweite Spülflüssigkeitseintrittsöffnung 18 andererseits sind dadurch zur Ausbildung zweier durch den Kragen 4 getrennter Kreisläufe von medizinischer Spülflüssigkeit entlang der distalen und der proximalen Oberflächen des Hüftgelenkspacers geeignet.

Die Befestigungsflächen 6, 7 sind jeweils begrenzt durch je einen umlaufenden Steg 21, 22 und an den proximalen Seiten der Befestigungsflächen 6, 7 durch den Kragen 4. Die Stege 21, 22 erheben sich aus der Oberfläche des Schafts 5 und reichen bis an den Kragen 4. Die Stege 21, 22 sind als Teil des Prothesenkörpers zu verstehen. Durch die vorstehenden Stege 21, 22 und den Kragen 4 wird verhindert oder zumindest erschwert, dass beim Befestigen des Hüftgelenkspacers am Femur Knochenzementteig außerhalb der Befestigungsflächen 6, 7 vordringt und dadurch die erste Spülflüssigkeitsaustrittsöffnung 14, die erste Spülflüssigkeitseintrittsöffnung 16, die zweite Spülflüssigkeitsaustrittsöffnung 20, die zweite Spülflüssigkeitseintrittsöffnung 18 oder die Spülflüssigkeits-Einlassöffnung und die Spülflüssigkeits-Auslassöffnung verschließt oder beeinträchtigt beziehungsweise ungewollt das erste Verbindungsmittel 8 oder das zweite Verbindungsmittel 9 am Prothesenkörper festzementiert.

Das erste Verbindungsmittel 8 hat einen Luer-Lock-Adapter 24 und einen kurzen, flexiblen Schlauch 26. Ebenso hat das zweite Verbindungsmittel 9 einen Luer-Lock-Adapter 25 und einen kurzen, flexiblen Schlauch 27. Dadurch kann der Hüftgelenkspacer mit dem ersten Verbindungsmittel 8 über den Luer-Lock-Adapter 24 an eine Quelle für eine medizinische Spülflüssigkeit mit einer Pumpe (nicht gezeigt) angeschlossen werden und das zweite Verbindungsmittel 9 über den Luer-Lock-Adapter 25 an einen Sammelbehälter und gegebenenfalls ebenfalls eine Pumpe (nicht gezeigt).

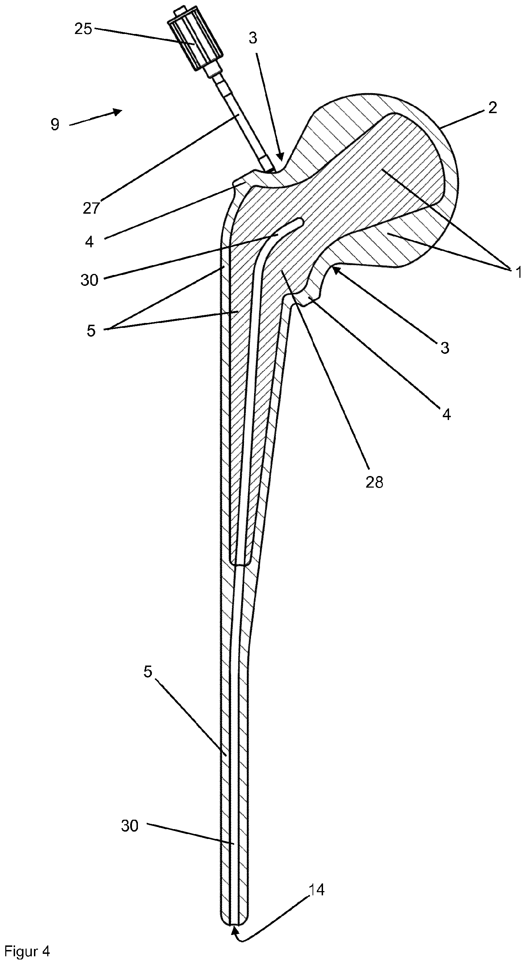

In den Querschnittansichten nach den Figuren 4, 5 und 7 ist zu erkennen, wie die die Spülflüssigkeits-Auslassöffnung mit der ersten Spülflüssigkeitseintrittsöffnung 16 und der zweiten Spülflüssigkeitseintrittsöffnung 18 und die Spülflüssigkeits-Einlassöffnung mit der ersten Spülflüssigkeitsaustrittsöffnung 14 und der zweiten Spülflüssigkeitsaustrittsöffnung 20 im Inneren des Prothesenkörpers verbunden sind. Zudem ist in den Querschnittansichten zu sehen, dass der Hüftgelenkspacer einen Kern 28 aus einem Metall aufweist, während die äußeren Bereiche des Prothesenkörpers im Wesentlichen aus einem Kunststoff gefertigt sind. Bevorzugt aus einem Knochenzement, wie einem PMMA-Kunststoff, der mit einem Antibiotikum oder mit mehreren Antibiotika beladen sein kann.

Im Inneren des Prothesenkörpers ist die Spülflüssigkeits-Einlassöffnung mit der ersten Spülflüssigkeitsaustrittsöffnung 14 und der zweiten Spülflüssigkeitsaustrittsöffnung 20 über eine erste Leitung 30 verbunden. Die Leitung 30 stellt eine flüssigkeitsdurchlässige Verbindung zwischen der Spülflüssigkeits-Einlassöffnung und der ersten Spülflüssigkeitsaustrittsöffnung 14 und der zweiten Spülflüssigkeitsaustrittsöffnung 20 her. Hierzu ist im Inneren des Prothesenkörpers im Bereich des Querschnitts am Hals 3 ein Abzweig in Form eines T-Stücks in der ersten Leitung 30 vorhanden. Ebenso ist im Inneren des Prothesenkörpers die Spülflüssigkeits-Auslassöffnung mit der ersten Spülflüssigkeitseintrittsöffnung 16 und der zweiten Spülflüssigkeitseintrittsöffnung 18 über eine zweite Leitung 32 verbunden. Auch die zweite Leitung 32 umfasst zu diesem Zweck einen Abzweig. Die erste Leitung 30 und die zweite Leitung 32 sind im Inneren des Prothesenkörpers voneinander getrennt.

Unmittelbar vor der Spülflüssigkeits-Auslassöffnung ist in der zweiten Leitung 32 ein Ventilelement 34 vorgesehen, das einen Abfluss von Flüssigkeit aus der zweiten Leitung 32 durch die Spülflüssigkeits-Auslassöffnung aus dem Prothesenkörper in das zweite Verbindungsmittel 9 erlaubt und das einen Rückfluss aus dem zweiten Verbindungsmittel 9 in die zweite Leitung 32 hinein verhindert. Das zweite Verbindungsmittel 9 ist über ein lösbares Verbindungselement 36 mit der Spülflüssigkeits-Auslassöffnung verbunden.

Unmittelbar vor der Spülflüssigkeits-Einlassöffnung ist in der ersten Leitung 30 ein Ventilelement 38 vorgesehen, dass einen Zufluss der medizinischen Spülflüssigkeit in die erste Leitung 30 durch die Spülflüssigkeits-Einlassöffnung in den Prothesenkörper hinein erlaubt und das einen Rückfluss aus der ersten Leitung 30 in das erste Verbindungsmittel 8 verhindert. Das erste Verbindungsmittel 8 ist über ein lösbares Verbindungselement 40 mit der Spülflüssigkeits-Einlassöffnung verbunden.

Das erste Verbindungsmittel 8 und das zweite Verbindungsmittel 9 können durch abziehen oder abschrauben der lösbaren Verbindungselemente 36, 40 von dem Prothesenkörper gelöst werden. Hierzu sind flüssigkeitsdurchlässige Gegenbefestigungselemente 42, 44 in den Leitungen 30, 32 im Prothesenkörper vorgesehen. Die Gegenbefestigungselemente 42, 44 können beispielsweise durch Hülsen mit Innengewinden realisiert werden, in die die Verbindungselemente 36, 40 in Form von flüssigkeitsdurchlässigen Hülsen mit Außengewinden eingeschraubt sind oder einschraubbar sind.

Im eingesetzten Zustand kann der femorale Hüftgelenkspacer wie folgt zum Spülen verwendet werden: Durch das erste Verbindungsmittel 8 wird eine medizinische Spülflüssigkeit mit einer an die Bedürfnisse des Patienten angepassten Zusammensetzung, wie beispielsweise eine sterile Ringer-Lösung mit einer Mischung geeigneter Antibiotika, in den Prothesenkörper eingespeist. Die medizinische Spülflüssigkeit fließt durch das Ventilelement 38 und durch die erste Leitung 30 durch den Prothesenkörper und tritt durch die erste Spülflüssigkeitsaustrittsöffnung 14 und durch die zweite Spülflüssigkeitsaustrittsöffnung 20 aus dem Prothesenkörper aus. Die Spülflüssigkeit fließt anschließend entlang der Oberfläche des Hüftgelenkspacers von der ersten Spülflüssigkeitsaustrittsöffnung 14 zu der ersten Spülflüssigkeitseintrittsöffnung 16 und von der zweiten Spülflüssigkeitsaustrittsöffnung 20 zu der zweiten Spülflüssigkeitseintrittsöffnung 18. Die Bereiche dazwischen werden mit einer Schicht der medizinischen Spülflüssigkeit gespült. Die gebrauchte Spülflüssigkeit tritt an der ersten Spülflüssigkeitseintrittsöffnung 16 und der zweiten Spülflüssigkeitseintrittsöffnung 18 wieder in den Prothesenkörper ein und fließt durch die zweite Leitung 32 und das Ventilelement 38 zur Spülflüssigkeits-Auslassöffnung. Von dort wird es durch das zweite Verbindungsmittel 9 aus dem Prothesenkörper abgesaugt und die gebrauchte Spülflüssigkeit entsorgt oder gesammelt.

Wenn keine Spülung mehr erfolgen soll, können die Verbindungsmittel 8, 9 mit den Verbindungselementen 36, 40 von dem Prothesenkörper getrennt werden und der restliche Hüftgelenkspacer kann wie ein normaler Hüftgelenkspacer auch verwendet werden. Es kann bevorzugt vorgesehen sein, dass sich die Spülflüssigkeits-Einlassöffnung und die Spülflüssigkeits-Auslassöffnung beim Abziehen oder Abschrauben der Verbindungselemente 36, 40 von dem Prothesenkörper selbsttätig verschließen.

Als Variante des ersten beispielhaften Hüftgelenkspacers könnten die distale Spülflüssigkeitseintrittsöffnung 16 und die proximale Spülflüssigkeitsaustrittsöffnung 20 verschlossen sein. Dadurch würde nur ein einziger Kreislauf der Spülflüssigkeit über die distale Spülflüssigkeitsaustrittsöffnung 14 und die proximale Spülflüssigkeitseintrittsöffnung 18 erzeugt werden, der sich sowohl über die distale als auch über die proximale Seite des Hüftgelenkspacers erstreckt. Die Spülflüssigkeit muss dann über den Kragen 4 fließen.

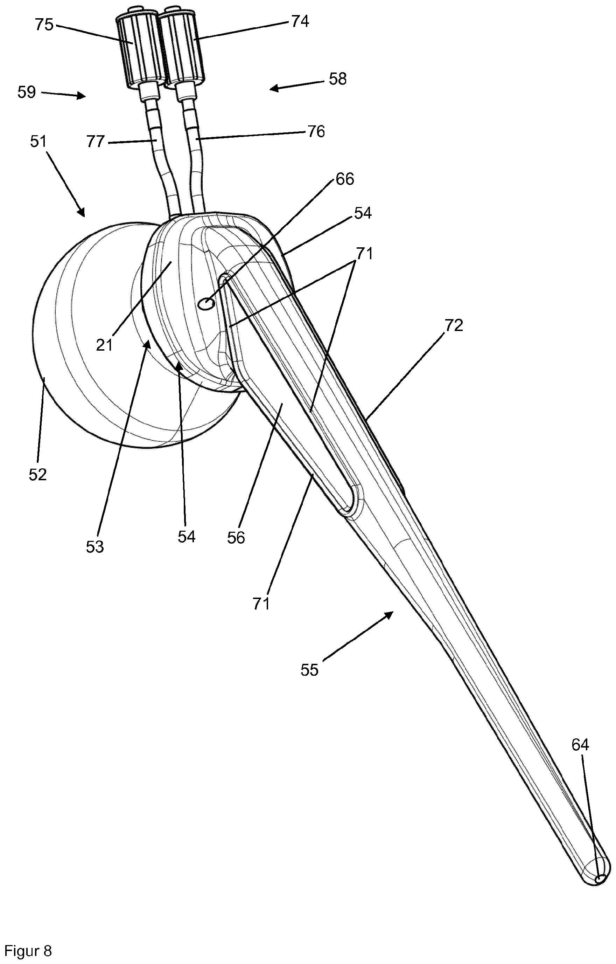

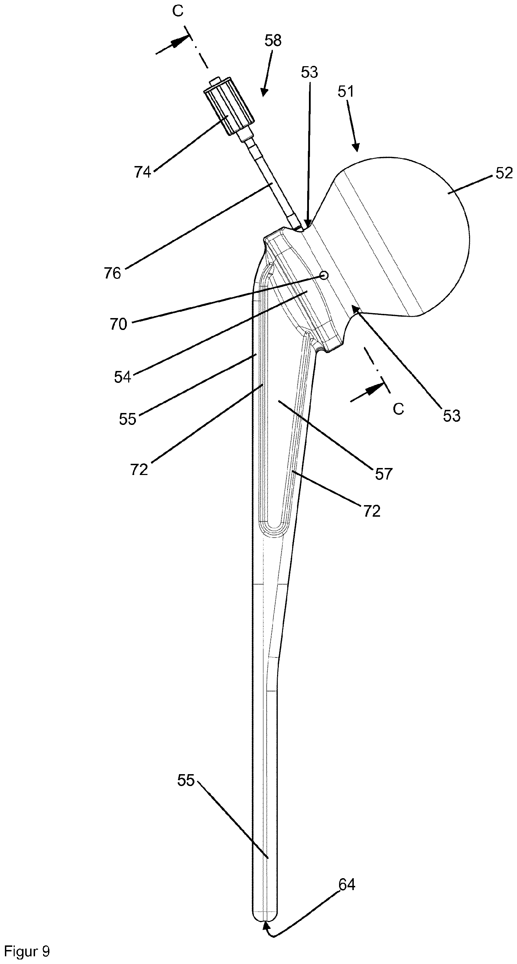

In den Figuren 8 bis 12 sind Abbildungen eines zweiten Ausführungsbeispiels eines erfindungsgemäßen Hüftgelenkspacers mit Spülvorrichtung gezeigt. Der femorale Hüftgelenkspacer weist einen Kugelkopf 51 mit einer Gleitfläche 52 an der proximalen Seite auf. Die Gleitfläche 52 liegt im eingesetzten Zustand an der Hüftgelenkspfanne an und bildet so einen Teil des Hüftgelenks. Der Kugelkopf 51 ist auf der der Gleitfläche 52 gegenüberliegenden distalen Seite über einen Hals 53 mit einem Kragen 54 verbunden. Der Hals 53 ist dünner als der Kugelkopf 51 und der Kragen 54. An der distalen Seite des Kragens 54 ist ein Schaft 55 befestigt, der sich in die distale Richtung erstreckt und der zur Befestigung des Hüftgelenkspacers im Femur dient. Hierzu weist der Hüftgelenkspacer an zwei gegenüberliegenden Seiten des Schafts 55 Befestigungsflächen 56, 57 auf, die zur Verbindung des Hüftgelenkspacers mit dem Femur mit Hilfe von Knochenzementteig vorgesehen sind. Der Kugelkopf 51, der Hals 53, der Kragen 54 und der Schaft 55 bilden einen Prothesenkörper des Hüftgelenkspacers. Der Prothesenkörper entspricht insoweit in seiner äußeren Form bis auf die geringere Fläche einer Befestigungsfläche 56 dem ersten beispielhaften Hüftgelenkspacer nach den Figuren 1 bis 7.

An einer Seite des zweiten beispielhaften Hüftgelenkspacers sind ein erstes röhrenförmiges Verbindungsmittel 58 an einer Spülflüssigkeits-Einlassöffnung befestigt und ein zweites röhrenförmiges Verbindungsmittel 59 an einer Spülflüssigkeits-Auslassöffnung befestigt. Die Spülflüssigkeits-Einlassöffnung und die Spülflüssigkeits-Auslassöffnung führen ins Innere des Prothesenkörpers und sind im Bereich des Halses 53 angeordnet. Das erste röhrenförmige Verbindungsmittel 58 und das zweite röhrenförmige Verbindungsmittel 59 sind flüssigkeitsdurchlässig, so dass eine medizinische Spülflüssigkeit durch das erste röhrenförmige Verbindungsmittel 58 in den Prothesenkörper hineingeleitet werden kann und eine Flüssigkeit durch das zweite röhrenförmige Verbindungsmittel 59 aus dem Prothesenkörper abgeführt werden kann. Das erste Verbindungsmittel 58 und das zweite Verbindungsmittel 59 sind lösbar mit der Spülflüssigkeits-Einlassöffnung und der Spülflüssigkeits-Auslassöffnung verbunden.

Am distalen Ende des Schafts 55 ist eine erste Spülflüssigkeitsaustrittsöffnung 64 angeordnet und am Übergang vom Schaft 55 zum Kragen 54 ist eine erste Spülflüssigkeitseintrittsöffnung 66 im Bereich zwischen einer der Befestigungsflächen 56 und dem Kragen 54 angeordnet. Des Weiteren ist am Hals 53 beziehungsweise am Übergang vom Hals 53 zum Kragen 54 eine zweite Spülflüssigkeitseintrittsöffnung 68 und auf der zur zweiten Spülflüssigkeitseintrittsöffnung 68 gegenüberliegenden Seite am Hals 53 beziehungsweise am Übergang vom Hals 53 zum Kragen 54 eine zweite Spülflüssigkeitsaustrittsöffnung 70 angeordnet. Die erste Spülflüssigkeitsaustrittsöffnung 64 und die erste Spülflüssigkeitseintrittsöffnung 66 sind dadurch auf der distalen Seite des Hüftgelenkspacers angeordnet und die zweite Spülflüssigkeitsaustrittsöffnung 70 und die zweite Spülflüssigkeitseintrittsöffnung 68 sind auf der proximalen Seite des Hüftgelenkspacers angeordnet, wobei die proximale und die distale Seite durch den Kragen 54 getrennt sind. Die erste Spülflüssigkeitsaustrittsöffnung 64 und die erste Spülflüssigkeitseintrittsöffnung 66 einerseits und die zweite Spülflüssigkeitsaustrittsöffnung 70 und die zweite Spülflüssigkeitseintrittsöffnung 68 andererseits sind dadurch zur Ausbildung zweier durch den Kragen 54 getrennter Kreisläufe von medizinischer Spülflüssigkeit entlang der distalen und der proximalen Oberflächen des Hüftgelenkspacers geeignet.

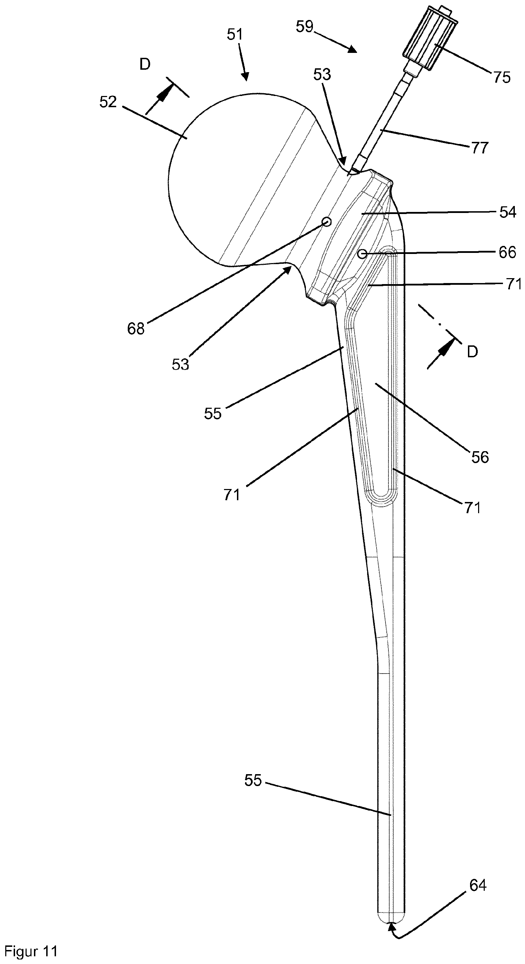

Im Unterschied zur ersten beispielhaften Ausführung nach den Figuren 1 bis 7 ist eine Befestigungsfläche 56 vollständig durch einen umlaufenden Steg 71 begrenzt, während die andere Befestigungsfläche 57 analog dem ersten Ausführungsbeispiel begrenzt ist durch einen umlaufenden Steg 72 und an der proximalen Seite der Befestigungsfläche 57 durch den Kragen 54. Die Stege 71, 72 erheben sich aus der Oberfläche des Schafts 55. Die Stege 71, 72 sind als Teil des Prothesenkörpers zu verstehen. Durch die vorstehenden Stege 71, 72 und den Kragen 54 wird verhindert oder zumindest erschwert, dass beim Befestigen des Hüftgelenkspacers am Femur Knochenzementteig außerhalb der Befestigungsflächen 56, 57 vordringt und dadurch die erste Spülflüssigkeitsaustrittsöffnung 64, die erste Spülflüssigkeitseintrittsöffnung 66, die zweite Spülflüssigkeitsaustrittsöffnung 70, die zweite Spülflüssigkeitseintrittsöffnung 68 oder die Spülflüssigkeits-Einlassöffnung und die Spülflüssigkeits-Auslassöffnung verschließt oder beeinträchtigt beziehungsweise ungewollt das erste Verbindungsmittel 58 oder das zweite Verbindungsmittel 59 am Prothesenkörper festzementiert. Die zweite Spülflüssigkeitseintrittsöffnung 66 ist abweichend von der ersten beispielhaften Ausführungsform zwischen dem Kragen 54 und der Befestigungsfläche 56 angeordnet (wie in Figur 11 gut zu erkennen ist).

Das erste Verbindungsmittel 58 hat einen Luer-Lock-Adapter 74 und einen kurzen, flexiblen Schlauch 76. Ebenso hat das zweite Verbindungsmittel 59 einen Luer-Lock-Adapter 75 und einen kurzen, flexiblen Schlauch 77. Dadurch kann der Hüftgelenkspacer mit dem ersten Verbindungsmittel 58 über den Luer-Lock-Adapter 74 an eine Quelle für eine medizinische Spülflüssigkeit mit Pumpe (nicht gezeigt) angeschlossen werden und das zweite Verbindungsmittel 59 über den Luer-Lock-Adapter 76 an einen Sammelbehälter und gegebenenfalls ebenfalls eine Pumpe (nicht gezeigt).

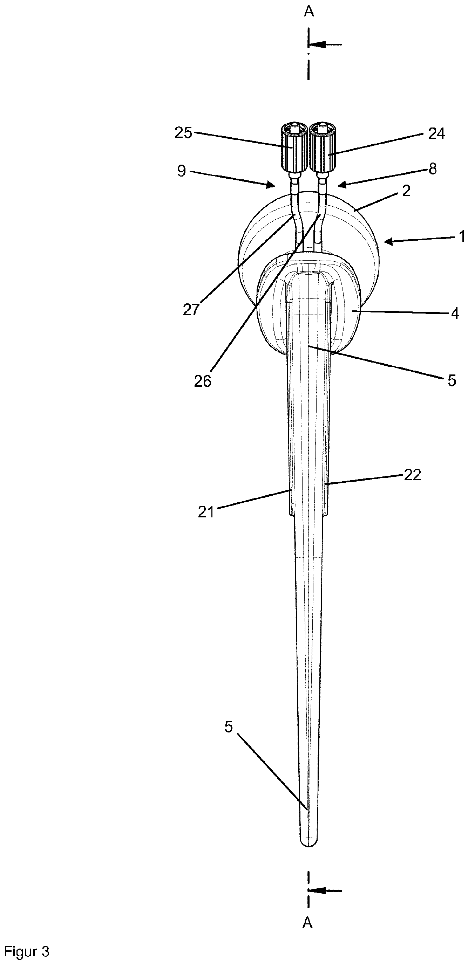

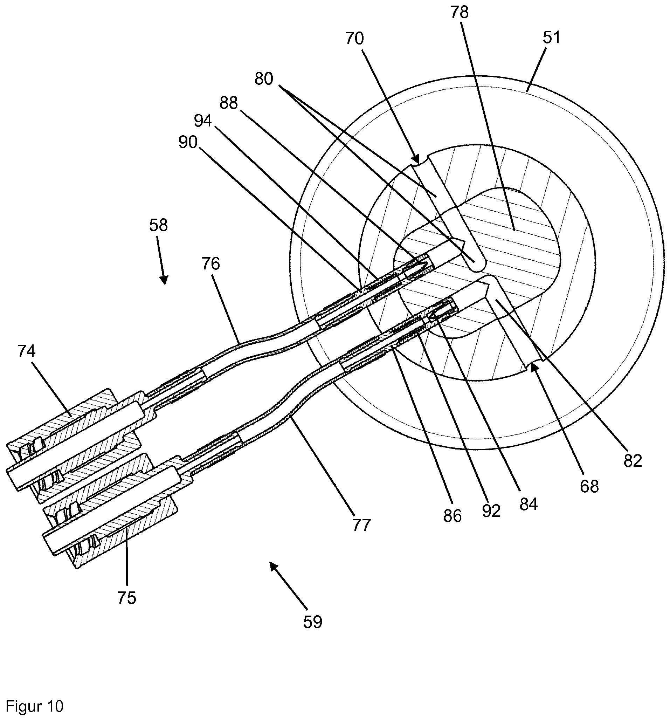

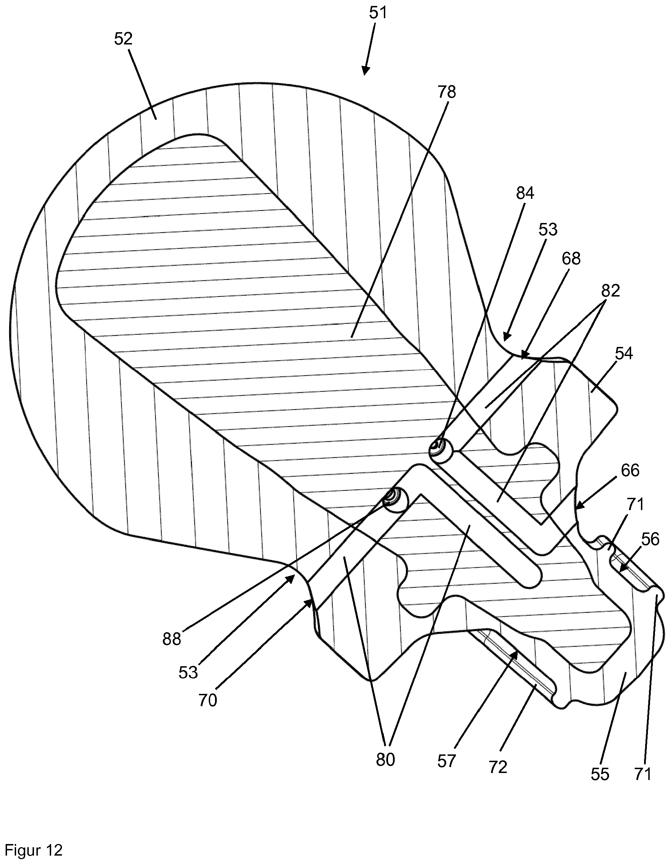

In den Querschnittansichten nach den Figuren 10 und 12 ist zu erkennen, wie die die Spülflüssigkeits-Auslassöffnung mit der ersten Spülflüssigkeitseintrittsöffnung 66 und der zweiten Spülflüssigkeitseintrittsöffnung 68 und die Spülflüssigkeits-Einlassöffnung mit der ersten Spülflüssigkeitsaustrittsöffnung 64 und der zweiten Spülflüssigkeitsaustrittsöffnung 70 im Inneren des Prothesenkörpers verbunden sind. Bei einem Schnitt analog dem Schnitt A in Figur 3 ergäbe sich bei dem zweiten beispielhaften Hüftgelenkspacer ein Querschnitt analog der Figur 4. in den Querschnittansichten ist auch zu sehen, dass der Hüftgelenkspacer einen Kern 78 aus einem Metall aufweist, während die äußeren Bereiche des Prothesenkörpers im Wesentlichen aus einem Kunststoff gefertigt sind. Bevorzugt aus einem Knochenzement, wie einem PMMA-Kunststoff, der mit einem Antibiotikum oder mit mehreren Antibiotika beladen sein kann.

Im Inneren des Prothesenkörpers ist die Spülflüssigkeits-Einlassöffnung mit der ersten Spülflüssigkeitsaustrittsöffnung 64 und der zweiten Spülflüssigkeitsaustrittsöffnung 70 über eine erste Leitung 80 verbunden. Die Leitung 80 stellt eine flüssigkeitsdurchlässige Verbindung zwischen der Spülflüssigkeits-Einlassöffnung und der ersten Spülflüssigkeitsaustrittsöffnung 64 und der zweiten Spülflüssigkeitsaustrittsöffnung 70 her. Hierzu ist im Inneren des Prothesenkörpers im Bereich des Querschnitts am Hals 53 ein Abzweig in Form eines T-Stücks in der ersten Leitung 80 vorhanden. Ebenso ist im Inneren des Prothesenkörpers die Spülflüssigkeits-Auslassöffnung mit der ersten Spülflüssigkeitseintrittsöffnung 66 und der zweiten Spülflüssigkeitseintrittsöffnung 68 über eine zweite Leitung 82 verbunden. Auch die zweite Leitung 82 umfasst zu diesem Zweck einen Abzweig. Die erste Leitung 80 und die zweite Leitung 82 sind im Inneren des Prothesenkörpers voneinander getrennt.

Unmittelbar vor der Spülflüssigkeits-Auslassöffnung ist in der zweiten Leitung 82 ein Ventilelement 84 vorgesehen, das einen Abfluss von Flüssigkeit aus der zweiten Leitung 82 durch die Spülflüssigkeits-Auslassöffnung aus dem Prothesenkörper in das zweite Verbindungsmittel 59 erlaubt und das einen Rückfluss aus dem zweiten Verbindungsmittel 59 in die zweite Leitung 82 hinein verhindert. Das zweite Verbindungsmittel 59 ist über ein lösbares Verbindungselement 86 mit der Spülflüssigkeits-Auslassöffnung verbunden.

Unmittelbar vor der Spülflüssigkeits-Einlassöffnung ist in der ersten Leitung 80 ein Ventilelement 88 vorgesehen, dass einen Zufluss der medizinischen Spülflüssigkeit in die erste Leitung 80 durch die Spülflüssigkeits-Einlassöffnung in den Prothesenkörper hinein erlaubt und das einen Rückfluss aus der ersten Leitung 80 in das erste Verbindungsmittel 58 verhindert. Das erste Verbindungsmittel 58 ist über ein lösbares Verbindungselement 90 mit der Spülflüssigkeits-Einlassöffnung verbunden.

Das erste Verbindungsmittel 58 und das zweite Verbindungsmittel 59 können durch abziehen oder abschrauben der lösbaren Verbindungselemente 86, 90 von dem Prothesenkörper gelöst werden. Hierzu sind flüssigkeitsdurchlässige Gegenbefestigungselemente 92, 94 in den Leitungen 80, 82 im Prothesenkörper vorgesehen. Die Gegenbefestigungselemente 92, 94 können beispielsweise durch Hülsen mit Innengewinden realisiert werden, in die die Verbindungselemente 86, 90 in Form von flüssigkeitsdurchlässigen Hülsen mit Außengewinden eingeschraubt sind oder einschraubbar sind.

Im eingesetzten Zustand kann der femorale Hüftgelenkspacer wie folgt zum Spülen verwendet werden: Durch das erste Verbindungsmittel 58 wird eine medizinische Spülflüssigkeit mit einer an die Bedürfnisse des Patienten angepassten Zusammensetzung, wie beispielsweise eine sterile Ringer-Lösung mit einer Mischung geeigneter Antibiotika, in den Prothesenkörper eingespeist. Die medizinische Spülflüssigkeit fließt durch das Ventilelement 88 und durch die erste Leitung 80 durch den Prothesenkörper und tritt durch die erste Spülflüssigkeitsaustrittsöffnung 64 und durch die zweite Spülflüssigkeitsaustrittsöffnung 70 aus dem Prothesenkörper aus. Die Spülflüssigkeit fließt anschließend entlang der Oberfläche des Hüftgelenkspacers von der ersten Spülflüssigkeitsaustrittsöffnung 64 zu der ersten Spülflüssigkeitseintrittsöffnung 66 und von der zweiten Spülflüssigkeitsaustrittsöffnung 70 zu der zweiten Spülflüssigkeitseintrittsöffnung 68. Dabei umfließt die Spülflüssigkeit die durch den Steg 71 abgetrennte Befestigungsfläche 56. Die Bereiche dazwischen werden mit einer Schicht der medizinischen Spülflüssigkeit gespült. Die gebrauchte Spülflüssigkeit tritt an der ersten Spülflüssigkeitseintrittsöffnung 66 und der zweiten Spülflüssigkeitseintrittsöffnung 68 wieder in den Prothesenkörper ein und fließt durch die zweite Leitung 82 und das Ventilelement 88 zur Spülflüssigkeits-Auslassöffnung. Von dort wird es durch das zweite Verbindungsmittel 59 aus dem Prothesenkörper abgesaugt und die gebrauchte Spülflüssigkeit entsorgt oder gesammelt.

Wenn keine Spülung mehr erfolgen soll, können die Verbindungsmittel 58, 59 mit den Verbindungselementen 86, 90 von dem Prothesenkörper getrennt werden und der restliche Hüftgelenkspacer kann wie ein normaler Hüftgelenkspacer auch verwendet werden. Es kann bevorzugt vorgesehen sein, dass sich die Spülflüssigkeits-Einlassöffnung und die Spülflüssigkeits-Auslassöffnung beim Abziehen oder Abschrauben der Verbindungselemente 86, 90 von dem Prothesenkörper selbsttätig verschließen.

Als Variante des zweiten beispielhaften Hüftgelenkspacers könnten die distale Spülflüssigkeitseintrittsöffnung 66 und die proximale Spülflüssigkeitsaustrittsöffnung 70 verschlossen sein. Dadurch würde nur ein einziger Kreislauf der Spülflüssigkeit über die distale Spülflüssigkeitsaustrittsöffnung 64 und die proximale Spülflüssigkeitseintrittsöffnung 68 erzeugt werden, der sich sowohl über die distale als auch über die proximale Seite des Hüftgelenkspacers erstreckt. Die Spülflüssigkeit muss dann über den Kragen 54 fließen.

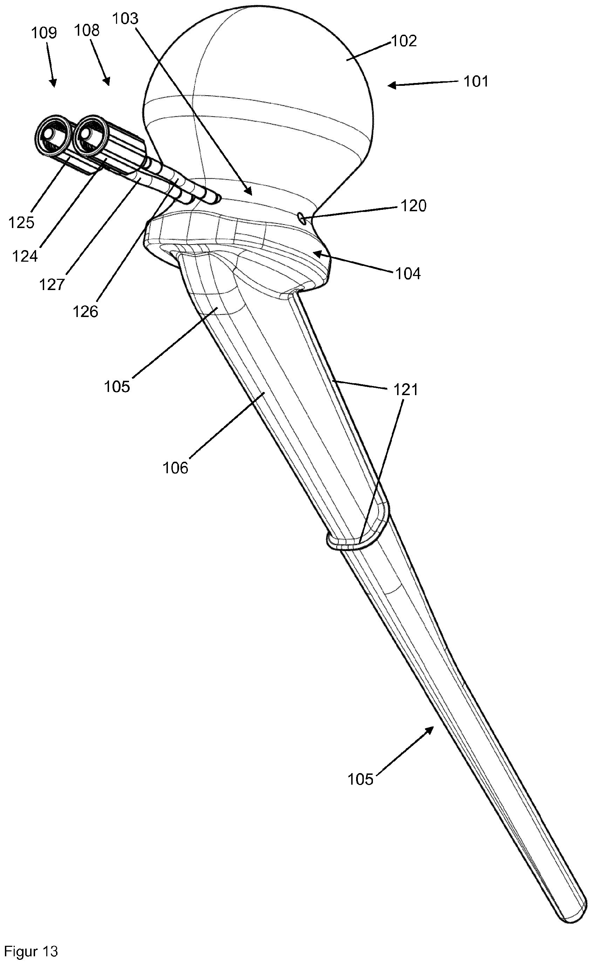

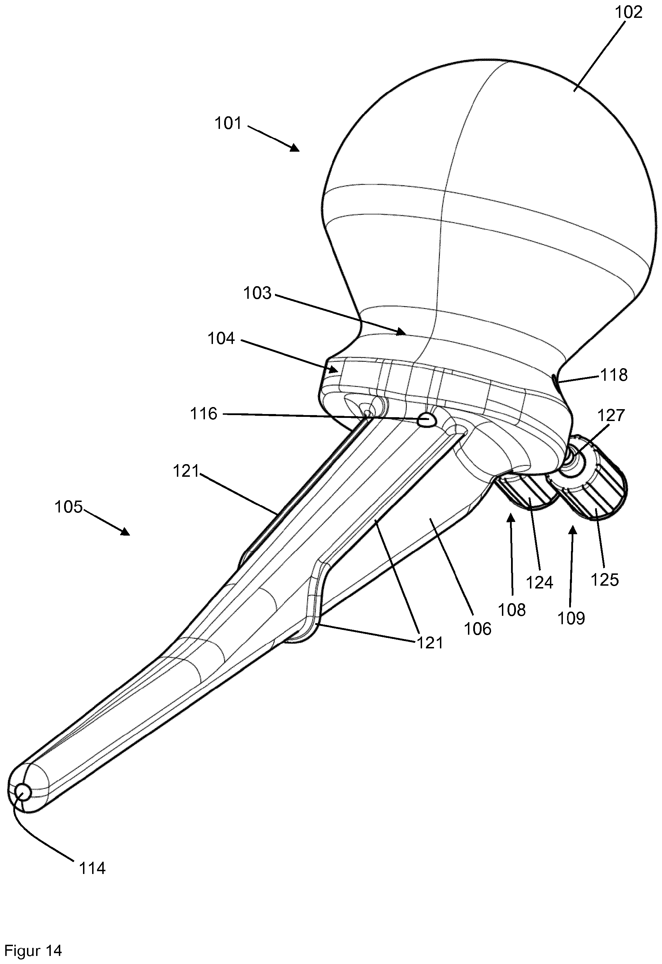

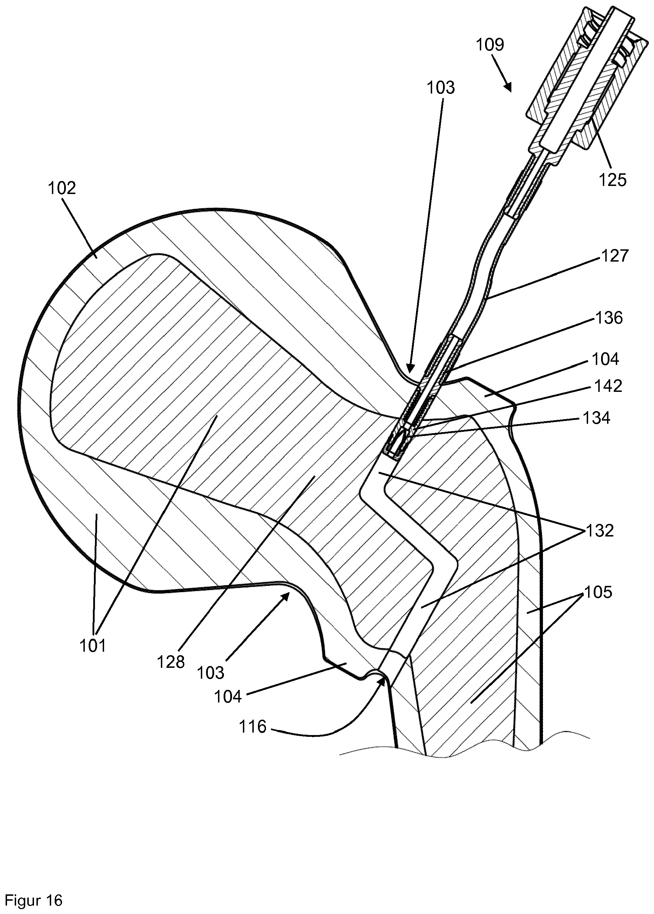

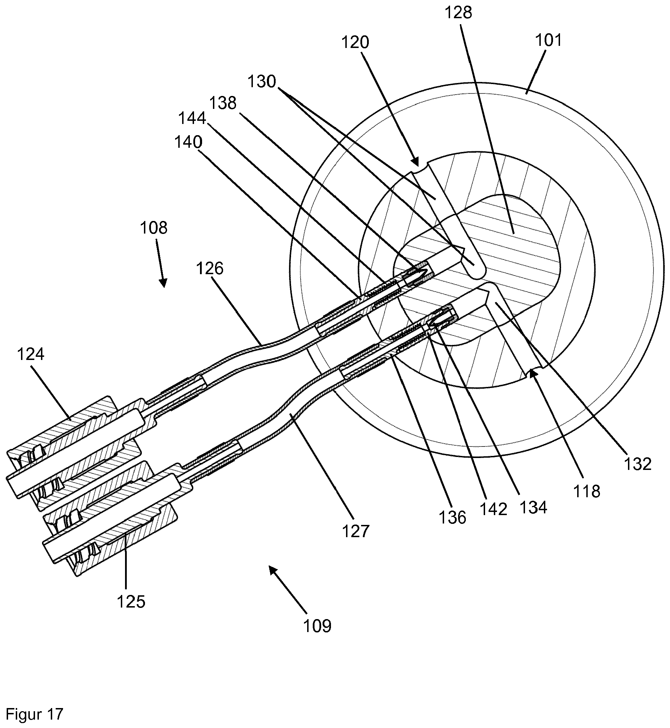

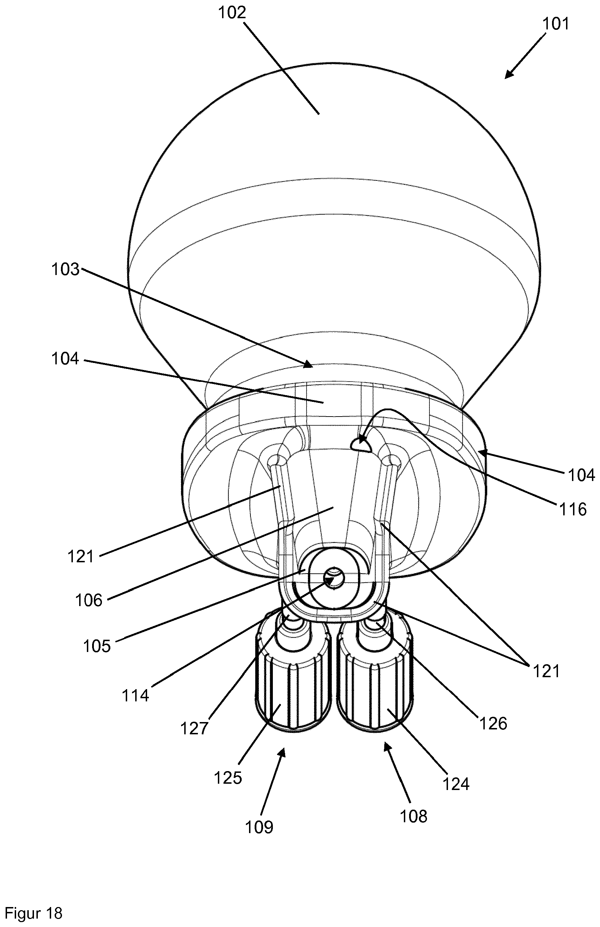

In den Figuren 13 bis 18 sind Abbildungen eines dritten Ausführungsbeispiels eines erfindungsgemäßen Hüftgelenkspacers mit Spülvorrichtung gezeigt. Der femorale Hüftgelenkspacer weist einen Kugelkopf 101 mit einer Gleitfläche 102 an der proximalen Seite auf. Die Gleitfläche 102 liegt im eingesetzten Zustand an der Hüftgelenkspfanne an und bildet so einen Teil des Hüftgelenks. Der Kugelkopf 101 ist auf der der Gleitfläche 102 gegenüberliegenden distalen Seite über einen Hals 103 mit einem Kragen 104 verbunden. Der Hals 103 ist dünner als der Kugelkopf 101 und der Kragen 104. An der distalen Seite des Kragens 104 ist ein Schaft 105 befestigt, der sich in die distale Richtung erstreckt und der zur Befestigung des Hüftgelenkspacers im Femur dient. Hierzu weist der Hüftgelenkspacer eine sich über mehrere Seiten des Schafts 105 erstreckende Befestigungsfläche 106 auf, die zur Verbindung des Hüftgelenkspacers mit dem Femur mit Hilfe von Knochenzementteig vorgesehen ist. Der Kugelkopf 101, der Hals 103, der Kragen 104 und der Schaft 105 bilden einen Prothesenkörper des Hüftgelenkspacers. Der Prothesenkörper entspricht insoweit in seiner äußeren Form bis auf die Form der Befestigungsfläche 106 und der Tatsache, dass nur eine Befestigungsfläche 106 vorhanden ist, dem ersten beispielhaften Hüftgelenkspacer nach den Figuren 1 bis 7 und dem zweiten beispielhaften Hüftgelenkspacer nach den Figuren 8 bis 12.

An einer Seite des dritten beispielhaften Hüftgelenkspacers sind im Unterschied zu bekannten Hüftgelenkspacern ein erstes röhrenförmiges Verbindungsmittel 108 an einer Spülflüssigkeits-Einlassöffnung befestigt und ein zweites röhrenförmiges Verbindungsmittel 109 an einer Spülflüssigkeits-Auslassöffnung befestigt. Die Spülflüssigkeits-Einlassöffnung und die Spülflüssigkeits-Auslassöffnung führen ins Innere des Prothesenkörpers und sind im Bereich des Halses 103 angeordnet. Das erste röhrenförmige Verbindungsmittel 108 und das zweite röhrenförmige Verbindungsmittel 109 sind flüssigkeitsdurchlässig, so dass eine medizinische Spülflüssigkeit durch das erste röhrenförmige Verbindungsmittel 108 in den Prothesenkörper hineingeleitet werden kann und eine Flüssigkeit durch das zweite röhrenförmige Verbindungsmittel 109 aus dem Prothesenkörper abgeführt werden kann. Das erste Verbindungsmittel 108 und das zweite Verbindungsmittel 109 sind lösbar mit der Spülflüssigkeits-Einlassöffnung und der Spülflüssigkeits-Auslassöffnung verbunden.

Am distalen Ende des Schafts 105 ist eine erste Spülflüssigkeitsaustrittsöffnung 114 angeordnet und am Übergang vom Schaft 105 zum Kragen 104 ist eine erste Spülflüssigkeitseintrittsöffnung 116 angeordnet. Des Weiteren ist am Hals 103 beziehungsweise am Übergang vom Hals 103 zum Kragen 104 eine zweite Spülflüssigkeitseintrittsöffnung 118 und auf der zur zweiten Spülflüssigkeitseintrittsöffnung 118 gegenüberliegenden Seite am Hals 103 beziehungsweise am Übergang vom Hals 103 zum Kragen 104 eine zweite Spülflüssigkeitsaustrittsöffnung 120 angeordnet. Die erste Spülflüssigkeitsaustrittsöffnung 114 und die erste Spülflüssigkeitseintrittsöffnung 116 sind dadurch auf der distalen Seite des Hüftgelenkspacers angeordnet und die zweite Spülflüssigkeitsaustrittsöffnung 120 und die zweite Spülflüssigkeitseintrittsöffnung 118 sind auf der proximalen Seite des Hüftgelenkspacers angeordnet, wobei die proximale und die distale Seite durch den Kragen 104 getrennt sind. Die erste Spülflüssigkeitsaustrittsöffnung 114 und die erste Spülflüssigkeitseintrittsöffnung 116 einerseits und die zweite Spülflüssigkeitsaustrittsöffnung 120 und die zweite Spülflüssigkeitseintrittsöffnung 118 andererseits sind dadurch zur Ausbildung zweier durch den Kragen 104 getrennter Kreisläufe von medizinischer Spülflüssigkeit entlang der distalen und der proximalen Oberflächen des Hüftgelenkspacers geeignet.

Die Befestigungsfläche 106 ist begrenzt durch einen umlaufenden Steg 121 und an der proximalen Seite der Befestigungsfläche 106 durch den Kragen 104. Der Steg 121 erhebt sich aus der Oberfläche des Schafts 105 und reicht an zwei gegenüberliegenden Seiten des Schafts 105 bis an den Kragen 104. Der Steg 121 ist als Teil des Prothesenkörpers zu verstehen. Durch den vorstehenden Stege 121 und den Kragen 104 wird verhindert oder zumindest erschwert, dass beim Befestigen des Hüftgelenkspacers am Femur Knochenzementteig außerhalb der Befestigungsfläche 106 vordringt und dadurch die erste Spülflüssigkeitsaustrittsöffnung 114, die erste Spülflüssigkeitseintrittsöffnung 116, die zweite Spülflüssigkeitsaustrittsöffnung 120, die zweite Spülflüssigkeitseintrittsöffnung 118 oder die Spülflüssigkeits-Einlassöffnung und die Spülflüssigkeits-Auslassöffnung verschließt oder beeinträchtigt beziehungsweise ungewollt das erste Verbindungsmittel 108 oder das zweite Verbindungsmittel 109 am Prothesenkörper festzementiert.

Das erste Verbindungsmittel 108 hat einen Luer-Lock-Adapter 124 und einen kurzen, flexiblen Schlauch 126. Ebenso hat das zweite Verbindungsmittel 109 einen Luer-Lock-Adapter 125 und einen kurzen, flexiblen Schlauch 127. Dadurch kann der Hüftgelenkspacer mit dem ersten Verbindungsmittel 108 über den Luer-Lock-Adapter 124 an eine Quelle für eine medizinische Spülflüssigkeit mit Pumpe (nicht gezeigt) angeschlossen werden und das zweite Verbindungsmittel 109 über den Luer-Lock-Adapter 126 an einen Sammelbehälter und gegebenenfalls ebenfalls eine Pumpe (nicht gezeigt).

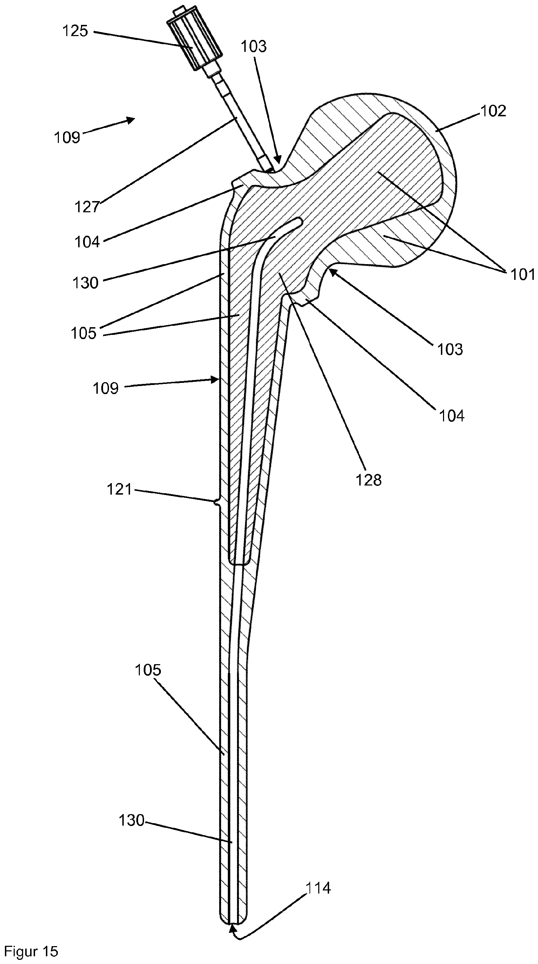

In den Querschnittansichten nach den Figuren 15, 16 und 17 ist zu erkennen, wie die die Spülflüssigkeits-Auslassöffnung mit der ersten Spülflüssigkeitseintrittsöffnung 116 und der zweiten Spülflüssigkeitseintrittsöffnung 118 und die Spülflüssigkeits-Einlassöffnung mit der ersten Spülflüssigkeitsaustrittsöffnung 114 und der zweiten Spülflüssigkeitsaustrittsöffnung 120 im Inneren des Prothesenkörpers verbunden sind. Zudem ist in den Querschnittansichten zu sehen, dass der Hüftgelenkspacer einen Kern 128 aus einem Metall aufweist, während die äußeren Bereiche des Prothesenkörpers im Wesentlichen aus einem Kunststoff gefertigt sind. Bevorzugt aus einem Knochenzement, wie einem PMMA-Kunststoff, der mit einem Antibiotikum oder mit mehreren Antibiotika beladen sein kann.

Im Inneren des Prothesenkörpers ist die Spülflüssigkeits-Einlassöffnung mit der ersten Spülflüssigkeitsaustrittsöffnung 114 und der zweiten Spülflüssigkeitsaustrittsöffnung 120 über eine erste Leitung 130 verbunden. Die Leitung 130 stellt eine flüssigkeitsdurchlässige Verbindung zwischen der Spülflüssigkeits-Einlassöffnung und der ersten Spülflüssigkeitsaustrittsöffnung 114 und der zweiten Spülflüssigkeitsaustrittsöffnung 120 her. Hierzu ist im Inneren des Prothesenkörpers im Bereich des Querschnitts am Hals 103 ein Abzweig in Form eines T-Stücks in der ersten Leitung 130 vorhanden. Ebenso ist im Inneren des Prothesenkörpers die Spülflüssigkeits-Auslassöffnung mit der ersten Spülflüssigkeitseintrittsöffnung 116 und der zweiten Spülflüssigkeitseintrittsöffnung 118 über eine zweite Leitung 132 verbunden. Auch die zweite Leitung 132 umfasst zu diesem Zweck einen Abzweig. Die erste Leitung 130 und die zweite Leitung 132 sind im Inneren des Prothesenkörpers voneinander getrennt.

Unmittelbar vor der Spülflüssigkeits-Auslassöffnung ist in der zweiten Leitung 132 ein Ventilelement 134 vorgesehen, das einen Abfluss von Flüssigkeit aus der zweiten Leitung 132 durch die Spülflüssigkeits-Auslassöffnung aus dem Prothesenkörper in das zweite Verbindungsmittel 109 erlaubt und das einen Rückfluss aus dem zweiten Verbindungsmittel 109 in die zweite Leitung 132 hinein verhindert. Das zweite Verbindungsmittel 109 ist über ein lösbares Verbindungselement 136 mit der Spülflüssigkeits-Auslassöffnung verbunden.

Unmittelbar vor der Spülflüssigkeits-Einlassöffnung ist in der ersten Leitung 130 ein Ventilelement 138 vorgesehen, dass einen Zufluss der medizinischen Spülflüssigkeit in die erste Leitung 130 durch die Spülflüssigkeits-Einlassöffnung in den Prothesenkörper hinein erlaubt und das einen Rückfluss aus der ersten Leitung 130 in das erste Verbindungsmittel 108 verhindert. Das erste Verbindungsmittel 108 ist über ein lösbares Verbindungselement 140 mit der Spülflüssigkeits-Einlassöffnung verbunden.

Das erste Verbindungsmittel 108 und das zweite Verbindungsmittel 109 können durch abziehen oder abschrauben der lösbaren Verbindungselemente 136, 140 von dem Prothesenkörper gelöst werden. Hierzu sind flüssigkeitsdurchlässige Gegenbefestigungselemente 142, 144 in den Leitungen 130, 132 im Prothesenkörper vorgesehen. Die Gegenbefestigungselemente 142, 144 können beispielsweise durch Hülsen mit Innengewinden realisiert werden, in die die Verbindungselemente 136, 140 in Form von flüssigkeitsdurchlässigen Hülsen mit Außengewinden eingeschraubt sind oder einschraubbar sind.

Im eingesetzten Zustand kann der femorale Hüftgelenkspacer wie folgt zum Spülen verwendet werden: Durch das erste Verbindungsmittel 108 wird eine medizinische Spülflüssigkeit mit einer an die Bedürfnisse des Patienten angepassten Zusammensetzung, wie beispielsweise eine sterile Ringer-Lösung mit einer Mischung geeigneter Antibiotika, in den Prothesenkörper eingespeist. Die medizinische Spülflüssigkeit fließt durch das Ventilelement 138 und durch die erste Leitung 130 durch den Prothesenkörper und tritt durch die erste Spülflüssigkeitsaustrittsöffnung 114 und durch die zweite Spülflüssigkeitsaustrittsöffnung 120 aus dem Prothesenkörper aus. Die Spülflüssigkeit fließt anschließend entlang der Oberfläche des Hüftgelenkspacers von der ersten Spülflüssigkeitsaustrittsöffnung 114 zu der ersten Spülflüssigkeitseintrittsöffnung 116 und von der zweiten Spülflüssigkeitsaustrittsöffnung 20 zu der zweiten Spülflüssigkeitseintrittsöffnung 118. Die Bereiche dazwischen werden mit einer Schicht der medizinischen Spülflüssigkeit gespült. Die gebrauchte Spülflüssigkeit tritt an der ersten Spülflüssigkeitseintrittsöffnung 116 und der zweiten Spülflüssigkeitseintrittsöffnung 118 wieder in den Prothesenkörper ein und fließt durch die zweite Leitung 132 und das Ventilelement 138 zur Spülflüssigkeits-Auslassöffnung. Von dort wird es durch das zweite Verbindungsmittel 109 aus dem Prothesenkörper abgesaugt und die gebrauchte Spülflüssigkeit entsorgt oder gesammelt.

Wenn keine Spülung mehr erfolgen soll, können die Verbindungsmittel 108, 109 mit den Verbindungselementen 136, 140 von dem Prothesenkörper getrennt werden und der restliche Hüftgelenkspacer kann wie ein normaler Hüftgelenkspacer auch verwendet werden. Es kann bevorzugt vorgesehen sein, dass sich die Spülflüssigkeits-Einlassöffnung und die Spülflüssigkeits-Auslassöffnung beim Abziehen oder Abschrauben der Verbindungselemente 136, 140 von dem Prothesenkörper selbsttätig verschließen.

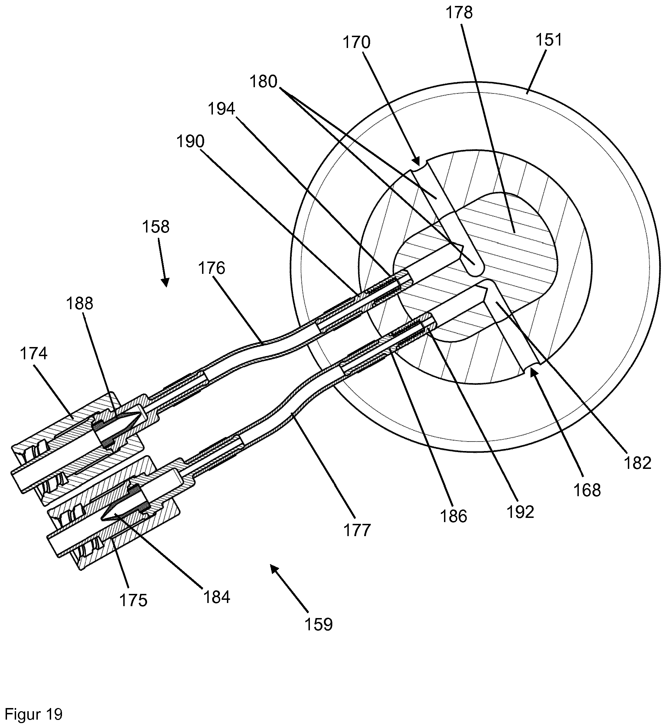

Als Variante des dritten beispielhaften Hüftgelenkspacers könnten die distale Spülflüssigkeitseintrittsöffnung 116 und die proximale Spülflüssigkeitsaustrittsöffnung 120 verschlossen sein. Dadurch würde nur ein einziger Kreislauf der Spülflüssigkeit über die distale Spülflüssigkeitsaustrittsöffnung 114 und die proximale Spülflüssigkeitseintrittsöffnung 118 erzeugt werden, der sich sowohl über die distale als auch über die proximale Seite des Hüftgelenkspacers erstreckt. Die Spülflüssigkeit muss dann über den Kragen 104 fließen. Figur 19 zeigt eine schematische Querschnittansicht durch eine Variante des ersten, zweiten, dritten und eines nachfolgenden vierten erfindungsgemäßen Hüftgelenkspacers der bis auf die Anordnung von Ventilelementen 184, 188 genauso wie der erste, zweite, dritte oder vierte erfindungsgemäße Hüftgelenkspacer aufgebaut ist. Die Ventilelemente 184, 188 sind nämlich nicht im Inneren des Prothesenkörpers angeordnet, sondern in zwei Verbindungsmitteln 158, 159 oder genauer in Luer-Lock-Adaptern 174, 175 der Verbindungsmittel 158, 159. Die Ventilelemente 184, 188 werden dann bei Bedarf zusammen mit den Verbindungsmitteln 186, 190 und den Schläuchen 176, 177 der Verbindungsmittel 158, 159 entfernt. Hierzu können die Verbindungsmittel 158, 159 aus passenden Gegenbefestigungsmitteln 192, 194 herausgeschraubt oder in anderer Weise von dem Prothesenkörper getrennt werden.

Da die in Figur 19 gezeigte Variante ansonsten bis auf die Anordnung der Ventilelemente 184, 188 identisch zur ersten, zweiten, dritten und der nachfolgenden vierten Ausführungsform gewählt werden kann, hat der Hüftgelenkspacer in diesen Varianten einen Kern 178 aus Metall, einen Kugelkopf 151 und Leitungen 180, 182 im Inneren des Prothesenkörpers. Ferner ist in Figur 19 die zweite Spülflüssigkeitseintrittsöffnung 168 und die zweite Spülflüssigkeitsaustrittsöffnung 170 zu sehen.

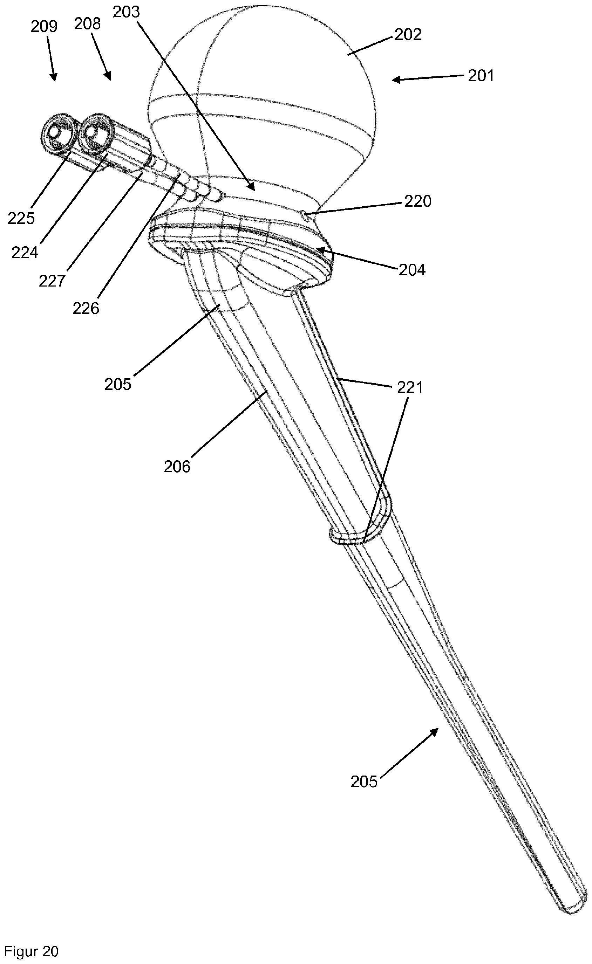

In den Figuren 20 bis 22 sind Abbildungen eines mehrteiligen vierten Ausführungsbeispiels eines erfindungsgemäßen Hüftgelenkspacers mit Spülvorrichtung gezeigt, der bis auf die Teilbarkeit dem dritten Ausführungsbeispiel nach den Figuren 13 bis 18 entspricht. Zur Vermeidung von Wiederholungen wird also für Details auf das dritte Ausführungsbeispiel verwiesen. Der femorale Hüftgelenkspacer weist dementsprechend einen Kugelkopf 201 mit einer Gleitfläche 202 an der proximalen Seite auf. Der Kugelkopf 201 ist auf der, der Gleitfläche 202 gegenüberliegenden distalen Seite über einen Hals 203 mit einem Kragen 204 verbunden. An der distalen Seite des Kragens 204 ist ein Schaft 205 befestigt, der sich in die distale Richtung erstreckt. Hierzu weist der Hüftgelenkspacer eine sich über mehrere Seiten des Schafts 205 erstreckende Befestigungsfläche 206 auf, die zur Verbindung des Hüftgelenkspacers mit dem Femur mit Hilfe von Knochenzementteig vorgesehen ist. Der Kugelkopf 201, der Hals 203, der Kragen 204 und der Schaft 205 bilden einen Prothesenkörper des Hüftgelenkspacers, wobei der Prothesenkörper in der Mitte des Kragens 204 trennbar ist, indem der Kopfteil (in den Figuren 20 bis 22 oben) von dem Schaftteil (in den Figuren 20 bis 22 unten) abschraubbar ist. Dadurch können unterschiedliche Kopfteile (nicht gezeigt) aufgeschraubt werden, die zur Anatomie des Patienten passen. Die unterschiedlichen Kopfteile unterscheiden sich dabei durch unterschiedliche Durchmesser des Kugelkopfs 201, so dass der Hüftgelenkspacer an Hüftgelenkpfannen unterschiedlicher Größe angepasst werden kann.

An einer Seite des vierten beispielhaften Hüftgelenkspacers sind ein erstes röhrenförmiges Verbindungsmittel 208 lösbar und flüssigkeitsdurchlässig an einer Spülflüssigkeits-Einlassöffnung befestigt und ein zweites röhrenförmiges Verbindungsmittel 209 lösbar und flüssigkeitsdurchlässig an einer Spülflüssigkeits-Auslassöffnung befestigt. Die Spülflüssigkeits-Einlassöffnung und die Spülflüssigkeits-Auslassöffnung führen ins Innere des Prothesenkörpers und sind im Bereich des Halses 203 angeordnet.

Am distalen Ende des Schafts 205 ist eine erste Spülflüssigkeitsaustrittsöffnung 214 angeordnet und am Übergang vom Schaft 205 zum Kragen 204 ist eine erste Spülflüssigkeitseintrittsöffnung 216 angeordnet. Des Weiteren ist am Hals 203 beziehungsweise am Übergang vom Hals 203 zum Kragen 204 eine zweite Spülflüssigkeitsaustrittsöffnung 220 angeordnet und auf der gegenüberliegenden Seite der zweiten Spülflüssigkeitsaustrittsöffnung 220 gegenüberliegenden Seite am Hals 203 eine zweite Spülflüssigkeitseintrittsöffnung (nicht zu sehen) angeordnet. Die erste Spülflüssigkeitsaustrittsöffnung 214 und die erste Spülflüssigkeitseintrittsöffnung 216 sind dadurch an dem distalen Schaftteil des Hüftgelenkspacers angeordnet und die zweite Spülflüssigkeitsaustrittsöffnung 220 und die zweite Spülflüssigkeitseintrittsöffnung sind an dem proximalen Kopfteil des zweiteiligen Hüftgelenkspacers angeordnet. Die erste Spülflüssigkeitsaustrittsöffnung 214 und die erste Spülflüssigkeitseintrittsöffnung 216 einerseits und die zweite Spülflüssigkeitsaustrittsöffnung 220 und die zweite Spülflüssigkeitseintrittsöffnung andererseits sind zur Ausbildung zweier durch den Kragen 204 getrennter Kreisläufe von medizinischer Spülflüssigkeit entlang der distalen und der proximalen Oberflächen des Hüftgelenkspacers geeignet.

Die Befestigungsfläche 206 ist begrenzt durch einen umlaufenden Steg 221 und an der proximalen Seite der Befestigungsfläche 206 durch den Kragen 204. Der Steg 221 erhebt sich aus der Oberfläche des Schafts 205 und reicht an zwei gegenüberliegenden Seiten des Schafts 205 bis an den Kragen 204 und ist als Teil des Prothesenkörpers zu verstehen.

Das erste Verbindungsmittel 208 hat einen Luer-Lock-Adapter 224 und einen kurzen, flexiblen Schlauch 226. Ebenso hat das zweite Verbindungsmittel 209 einen Luer-Lock-Adapter 225 und einen kurzen, flexiblen Schlauch 227.

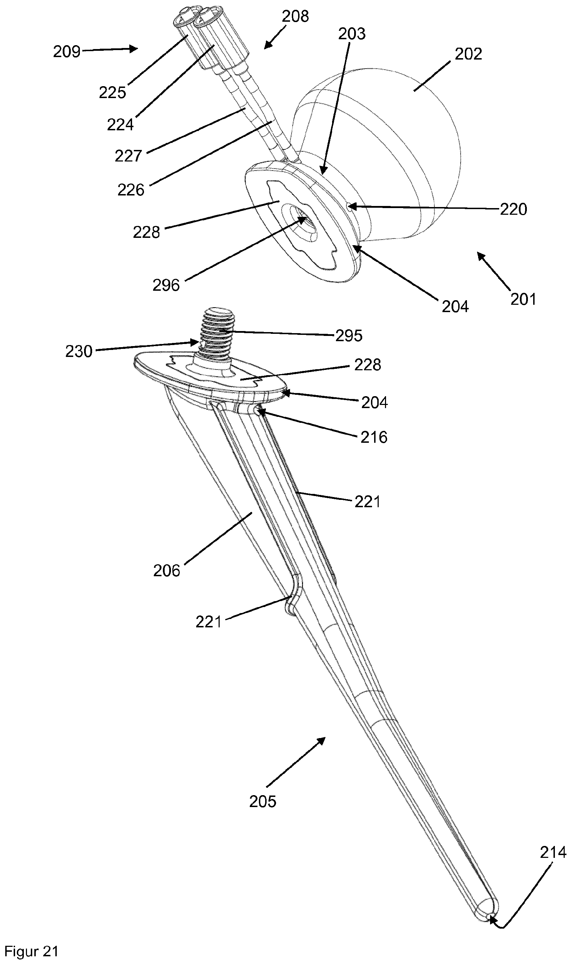

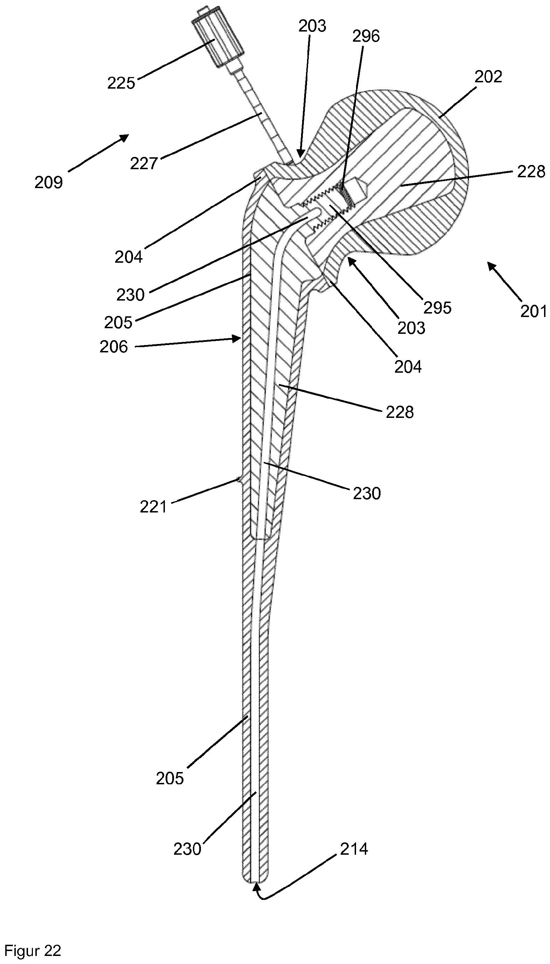

In der Querschnittansicht nach den Figur 22 ist angedeutet, dass die Spülflüssigkeits-Auslassöffnung mit der ersten Spülflüssigkeitseintrittsöffnung 216 und der zweiten Spülflüssigkeitseintrittsöffnung und die Spülflüssigkeits-Einlassöffnung mit der ersten Spülflüssigkeitsaustrittsöffnung 214 und der zweiten Spülflüssigkeitsaustrittsöffnung 220 wie beim dritten Ausführungsbeispiel im Inneren des Prothesenkörpers verbunden sind. Zudem ist in der Querschnittansicht nach Figur 20 zu sehen und in der getrennten Darstellung in Figur 21 zu erkennen, dass der Hüftgelenkspacer einen Kern 228 aus einem Metall aufweist, während die äußeren Bereiche des Prothesenkörpers im Wesentlichen aus einem Kunststoff gefertigt sind, das mit einer pharmazeutisch wirksamen Substanz versetzt sein kann.

Im Inneren des Prothesenkörpers ist die Spülflüssigkeits-Einlassöffnung mit der ersten Spülflüssigkeitsaustrittsöffnung 214 und der zweiten Spülflüssigkeitsaustrittsöffnung 220 über eine erste Leitung 230 verbunden. Die Leitung 230 stellt eine flüssigkeitsdurchlässige Verbindung zwischen der Spülflüssigkeits-Einlassöffnung und der ersten Spülflüssigkeitsaustrittsöffnung 214 und der zweiten Spülflüssigkeitsaustrittsöffnung 220 her. Ebenso ist im Inneren des Prothesenkörpers die Spülflüssigkeits-Auslassöffnung mit der ersten Spülflüssigkeitseintrittsöffnung 216 und der zweiten Spülflüssigkeitseintrittsöffnung über eine zweite Leitung (nicht zu sehen) verbunden. Die erste Leitung 230 und die zweite Leitung sind im Inneren des Prothesenkörpers voneinander getrennt.

Der Schaftteil und die unterschiedlichen Kopfteile können über eine Gewindestange 295 mit Außengewinde am Schaftteil und über eine Gewindebohrung 296 mit passendem Innengewinde im Kopfteil aneinandergeschraubt werden. Die Leitung 230 wird dabei durch die Gewindestange und die Gewinde geführt.

Alternativ ist es auch möglich, dass der mehrteilige Hüftgelenkspacer derart am Übergang vom Hals 203 zum Kugelkopf 201 oder am Kugelkopf 201 getrennt beziehungsweise trennbar ist, so dass die Leitungen 130 und alle Öffnungen 214, 216, 220 und die Verbindungsmittel 208, 209 am Schaftteil angeordnet sind. Dann muss kein Übergang der Leitung 230 an der Verbindung vom Kopfteil zum Schaftteil verwendet werden.

Die Verwendung von einem mit Antibiotika oder Antimykotika oder anderer pharmazeutisch wirksamer Substanzen versetztem PMMA zumindest als äußere Schicht erfindungsgemäßer Hüftgelenkspacer hat den Vorteil, dass initial großflächig eine besonders große Menge der Wirkstoffe zur Verfügung steht. Zudem ergibt sich ein besonderer kombinatorischer Effekt, dass nämlich der Kreislauf der medizinischen Spülflüssigkeit die Freisetzung der Wirkstoffe an der Oberfläche des Hüftgelenkspacers fördert und verstärkt.

### Bezugszeichenliste

- 1, 51, 101, 151, 201: Kugelkopf
- 2, 52, 102, 202: Gleitfläche
- 3, 53, 103, 203: Hals
- 4, 54, 104,204: Kragen
- 5, 55, 105,205: Schaft
- 6, 56, 106, 206: Befestigungsfläche
- 7, 57: Befestigungsfläche
- 8, 58, 108, 158, 208: Verbindungsmittel
- 9, 59, 109, 159, 209: Verbindungsmittel
- 14, 64, 114, 214: Spülflüssigkeitsaustrittsöffnung
- 16, 66, 116, 216: Spülflüssigkeitseintrittsöffnung
- 18, 68, 118, 168: Spülflüssigkeitseintrittsöffnung
- 20, 70, 120, 170, 220: Spülflüssigkeitsaustrittsöffnung
- 21, 71, 121, 221: Steg
- 22, 72: Steg
- 24, 74, 124, 174, 224: Luer-Lock-Adapter
- 25, 75, 125, 175, 225: Luer-Lock-Adapter
- 26, 76, 126, 176, 226: Schlauch
- 27, 77, 127, 177, 227: Schlauch
- 28, 78, 128, 178, 228: Kern
- 30, 80, 130, 180, 230: Leitung
- 32, 82, 132, 182: Leitung
- 34, 84, 134, 184: Ventilelement
- 36, 86, 136, 186: Verbindungselement
- 38, 88, 138, 188: Ventilelement
- 40, 90, 140, 190: Verbindungselement
- 42, 92, 142, 192: Gegenbefestigungselement
- 44, 94, 144, 194: Gegenbefestigungselement
- 295: Gewindestange
- 296: Gewindebohrung

## Patentansprüche

1. Femoraler Hüftgelenkspacer zum zeitweisen Ersetzen eines Teils eines Hüftgelenks, der Hüftgelenkspacer aufweisend
einen Prothesenkörper, der Prothesenkörper aufweisend einen Kugelkopf (1, 51, 101, 151, 201) mit einer Gleitfläche (2, 52, 102, 202), einen Hals (3, 53, 103, 203), der mit dem Kugelkopf (1, 51, 101, 151, 201) verbunden ist, einen Kragen (4, 54, 104, 204), der an einer proximalen Seite mit dem Hals (3, 53, 103, 203) verbunden ist, und einen Schaft (5, 55, 105, 205), der mit einer distalen Seite des Kragens (4, 54, 104, 204) verbunden ist, wobei zumindest eine Befestigungsfläche (6, 7, 56, 57, 106, 206) am Schaft (5, 55, 105, 205) des Prothesenkörpers angeordnet ist,
der Hüftgelenkspacer ferner aufweisend ein erstes röhrenförmiges und flüssigkeitsdurchlässiges Verbindungsmittel (8, 58, 108, 158, 208) zum Zuführen einer medizinischen Spülflüssigkeit in den Prothesenkörper,
eine Spülflüssigkeits-Einlassöffnung in einer Oberfläche des Prothesenkörpers, wobei das erste Verbindungsmittel (8, 58, 108, 158, 208) mit der Spülflüssigkeits-Einlassöffnung flüssigkeitsdurchlässig verbunden oder verbindbar ist,
zumindest eine Spülflüssigkeitsaustrittsöffnung (14, 20, 64, 70, 114, 120, 170, 214, 220), die in der Oberfläche des Prothesenkörpers außerhalb der zumindest einen Befestigungsfläche (6, 7, 56, 57, 106, 206) angeordnet ist, wobei die zumindest eine Spülflüssigkeitsaustrittsöffnung (14, 20, 64, 70, 114, 120, 170, 214, 220) im Inneren des Prothesenkörpers mit der Spülflüssigkeits-Einlassöffnung flüssigkeitsdurchlässig verbunden ist,
dadurch charakterisiert, dass der femoralen Hüftgelenkspacer ferner aufweist,
ein zweites röhrenförmiges und flüssigkeitsdurchlässiges Verbindungsmittel (9, 59, 109, 159, 209) zum Abführen der Spülflüssigkeit aus dem Prothesenkörper,
eine Spülflüssigkeits-Auslassöffnung in der Oberfläche des Prothesenkörpers, wobei das zweite Verbindungsmittel (9, 59, 109, 159, 209) mit der Spülflüssigkeits-Auslassöffnung flüssigkeitsdurchlässig verbunden oder verbindbar ist,
zumindest eine Spülflüssigkeitseintrittsöffnung (16, 18, 66, 68, 116, 118, 168, 216), die in der Oberfläche des Prothesenkörpers außerhalb der zumindest einen Befestigungsfläche (6, 7, 56, 57, 106, 206) angeordnet ist,
wobei die zumindest eine Spülflüssigkeitseintrittsöffnung (16, 18, 66, 68, 116, 118, 168, 216) im Inneren des Prothesenkörpers mit der Spülflüssigkeits-Auslassöffnung flüssigkeitsdurchlässig verbunden ist, wobei
die zumindest eine Spülflüssigkeitsaustrittsöffnung (14, 20, 64, 70, 114, 120, 170, 214, 220) im Inneren des Prothesenkörpers nicht flüssigkeitsdurchlässig mit der Spülflüssigkeits-Auslassöffnung verbunden ist und die zumindest eine Spülflüssigkeitseintrittsöffnung (16, 18, 66, 68, 116, 118, 168, 216) im Inneren des Prothesenkörpers nicht flüssigkeitsdurchlässig mit der Spülflüssigkeits-Einlassöffnung verbunden ist.

2. Hüftgelenkspacer nach Anspruch 1, **dadurch gekennzeichnet, dass** die zumindest eine Befestigungsfläche (6, 7, 56, 57, 106, 206) begrenzt ist oder die zumindest eine Befestigungsfläche (6, 7, 56, 57, 106, 206) mit einem umlaufenden, sich aus der Oberfläche des Prothesenkörpers erhebenden Steg (21, 22, 71, 72, 121, 221) begrenzt ist, so dass die zumindest eine Befestigungsfläche (6, 7, 56, 57, 106, 206) zur Aufnahme von Knochenzementteig innerhalb des Stegs (21, 22, 71, 72, 121, 221) geeignet ist, wobei vorzugsweise
wenigstens eine der zumindest einen Befestigungsfläche (6, 7, 56, 57, 106, 206) bereichsweise durch einen Teil des Kragens (4, 54, 104, 204) begrenzt ist.

3. Hüftgelenkspacer nach einem der vorangehenden Ansprüche, **dadurch gekennzeichnet, dass**
zumindest eine erste Spülflüssigkeitsaustrittsöffnung (14, 64, 114, 214) und zumindest eine erste Spülflüssigkeitseintrittsöffnung (16, 66, 116, 216) in der Oberfläche des Prothesenkörpers an der distalen Seite des Kragens (4, 54, 104, 204) oder am Schaft (5, 55, 105, 205) außerhalb der zumindest einen Befestigungsfläche (6, 7, 56, 57, 106, 206) angeordnet sind, und
zumindest eine zweite Spülflüssigkeitsaustrittsöffnung (20, 70, 120, 170, 220) und zumindest eine zweite Spülflüssigkeitseintrittsöffnung (18, 68, 118, 168) in der Oberfläche des Prothesenkörpers seitlich am Kragen (4, 54, 104, 204) oder an der proximalen Seite des Kragens (4, 54, 104, 204) oder am Hals (3, 53, 103, 203) oder am Kugelkopf (1, 51, 101, 151, 201) angeordnet sind, wobei
die zumindest eine erste Spülflüssigkeitsaustrittsöffnung (14, 64, 114, 214) und die zumindest eine zweite Spülflüssigkeitsaustrittsöffnung (20, 70, 120, 170, 220) im Inneren des Prothesenkörpers mit der Spülflüssigkeits-Einlassöffnung flüssigkeitsdurchlässig verbunden sind, und wobei
die zumindest eine erste Spülflüssigkeitseintrittsöffnung (16, 66, 116, 216) und die zumindest eine zweite Spülflüssigkeitseintrittsöffnung (18, 68, 118, 168) im Inneren des Prothesenkörpers mit der Spülflüssigkeits-Auslassöffnung flüssigkeitsdurchlässig verbunden sind.

4. Hüftgelenkspacer nach Anspruch 3, **dadurch gekennzeichnet, dass**
die zumindest eine erste Spülflüssigkeitsaustrittsöffnung (14, 64, 114, 214) und die zumindest eine erste Spülflüssigkeitseintrittsöffnung (16, 66, 116, 216) voneinander beabstandet sind und die zumindest eine zweite Spülflüssigkeitsaustrittsöffnung (20, 70, 120, 170, 220) und die zumindest eine zweite Spülflüssigkeitseintrittsöffnung (18, 68, 118, 168) voneinander beabstandet sind, wobei der Abstand zumindest 5 mm, bevorzugt zumindest 20 mm und besonders bevorzugt zumindest 30 mm beträgt, und/oder
die zumindest eine erste Spülflüssigkeitsaustrittsöffnung (14, 64, 114, 214) und die zumindest eine erste Spülflüssigkeitseintrittsöffnung (16, 66, 116, 216) durch zumindest einen umlaufenden und sich aus der Oberfläche des Prothesenkörpers erhebenden Steg (21, 22, 71, 72, 121, 221) von der zumindest einen Befestigungsfläche (6, 7, 56, 57, 106, 206) abgegrenzt sind.

5. Hüftgelenkspacer nach einem der Ansprüche 3 oder 4, **dadurch gekennzeichnet, dass** wenigstens eine Spülflüssigkeitsaustrittsöffnung (20, 70, 120, 170, 220) der zumindest einen zweiten Spülflüssigkeitsaustrittsöffnung (20, 70, 120, 170, 220) und wenigstens eine Spülflüssigkeitseintrittsöffnung (18, 68, 118, 168) der zumindest einen zweiten Spülflüssigkeitseintrittsöffnung (18, 68, 118, 168) neben der Gleitfläche (2, 52, 102, 202) am Kugelkopf (1, 51, 101, 151, 201) angeordnet sind, bevorzugt innerhalb von 5 mm neben der Gleitfläche (2, 52, 102, 202) am Kugelkopf (1, 51, 101, 151, 201) angeordnet sind, und/oder
wenigstens eine der zumindest einen ersten Spülflüssigkeitsaustrittsöffnung (14, 64, 114, 214) am Ende des Schafts (5, 55, 105, 205) angeordnet ist und wenigstens eine der zumindest einen ersten Spülflüssigkeitseintrittsöffnung (16, 66, 116, 216) an der distalen Seite des Kragens (4, 54, 104, 204), am Übergang vom Kragen (4, 54, 104, 204) zum Schaft (5, 55, 105, 205) oder an der proximalen Seite des Schafts (5, 55, 105, 205) angeordnet ist.

6. Hüftgelenkspacer nach einem der vorangehenden Ansprüche, **dadurch gekennzeichnet, dass**
die zumindest eine Spülflüssigkeitsaustrittsöffnung (14,20,64, 70, 114, 120, 170, 214, 220) und die zumindest eine Spülflüssigkeitseintrittsöffnung (16, 18, 66, 68, 116, 118, 168, 216) voneinander beabstandet sind, wobei der Abstand zumindest 5 mm, bevorzugt zumindest 20 mm und besonders bevorzugt zumindest 30 mm beträgt.

7. Hüftgelenkspacer nach einem der vorangehenden Ansprüche, **dadurch gekennzeichnet, dass**
das erste Verbindungsmittel (8, 58, 108, 158, 208) an der zur Verbindung mit der Spülflüssigkeits-Einlassöffnung abgewandten Seite und das zweite Verbindungsmittel (9, 59, 109, 159, 209) an der zur Verbindung mit der Spülflüssigkeits-Auslassöffnung abgewandten Seite jeweils einen Adapter (24, 25, 74, 75, 124, 125, 174, 175, 224, 225) aufweist, insbesondere jeweils einen Luer-Lock-Adapter (24, 25, 74, 75, 124, 125, 174, 175, 224, 225) aufweist, und/oder
an der Spülflüssigkeits-Einlassöffnung im Inneren des Prothesenkörpers oder an der Oberfläche des Prothesenkörpers eine selbstdichtende Kupplung (44, 94, 144, 194) angeordnet ist und an der Spülflüssigkeits-Auslassöffnung im Inneren des Prothesenkörpers oder an der Oberfläche des Prothesenkörpers eine selbstdichtende Kupplung (42, 92, 142, 192) angeordnet ist, wobei das erste Verbindungsmittel (8, 58, 108, 158, 208) lösbar mit der Spülflüssigkeits-Einlassöffnung verbunden oder verbindbar ist und das zweite Verbindungsmittel (9, 59, 109, 159, 209) lösbar mit der Spülflüssigkeits-Auslassöffnung verbunden oder verbindbar ist.

8. Hüftgelenkspacer nach einem der vorangehenden Ansprüche, **dadurch gekennzeichnet, dass**
die Spülflüssigkeits-Einlassöffnung eine erste Spülflüssigkeits-Einlassöffnung ist und die Spülflüssigkeits-Auslassöffnung eine erste Spülflüssigkeits-Auslassöffnung ist, wobei zusätzlich eine zweite Spülflüssigkeits-Einlassöffnung und eine zweite Spülflüssigkeits-Auslassöffnung in der Oberfläche des Prothesenkörpers vorgesehen sind, wobei an der ersten Spülflüssigkeits-Einlassöffnung, der zweiten Spülflüssigkeits-Einlassöffnung, der ersten Spülflüssigkeits-Auslassöffnung und der zweiten Spülflüssigkeits-Auslassöffnung jeweils eine selbstdichtende Kupplung (44, 46, 94, 96, 144, 146, 194, 196) angeordnet ist, wobei das erste Verbindungsmittel (8, 58, 108, 158, 208) flüssigkeitsdicht und lösbar mit der ersten Spülflüssigkeits-Einlassöffnung und mit der zweiten Spülflüssigkeits-Einlassöffnung verbindbar ist und das zweite Verbindungsmittel (9, 59, 109, 159, 209) flüssigkeitsdicht und lösbar mit der ersten Spülflüssigkeits-Auslassöffnung und mit der zweiten Spülflüssigkeits-Auslassöffnung verbindbar ist und wobei
die erste Spülflüssigkeits-Einlassöffnung und die zweite Spülflüssigkeits-Einlassöffnung im Prothesenkörper miteinander flüssigkeitsdurchlässig verbunden sind und die erste Spülflüssigkeits-Auslassöffnung und die zweite Spülflüssigkeits-Auslassöffnung im Prothesenkörper miteinander flüssigkeitsdurchlässig verbunden sind.

9. Hüftgelenkspacer nach einem der vorangehenden Ansprüche, **dadurch gekennzeichnet, dass**
die Summe der Querschnittsflächen aller der zumindest einen Spülflüssigkeitseintrittsöffnung (16, 18, 66, 68, 116, 118, 168, 216) zusammen zumindest genauso groß ist wie die Querschnittsfläche der Spülflüssigkeits-Einlassöffnung und/oder
die Summe der Querschnittsflächen aller der zumindest einen Spülflüssigkeitsaustrittsöffnung (14, 20, 64, 70, 114, 120, 170, 214, 220) zumindest genauso groß ist wie die Querschnittsfläche der Spülflüssigkeits-Auslassöffnung.

10. Hüftgelenkspacer nach einem der vorangehenden Ansprüche, **dadurch gekennzeichnet, dass**
die zumindest eine Spülflüssigkeitsaustrittsöffnung (14, 20, 64, 70, 114, 120, 170, 214, 220) und die zumindest eine Spülflüssigkeitseintrittsöffnung (16, 18, 66, 68, 116, 118, 168, 216) nicht in der Gleitfläche (2, 52, 102, 202) des Kugelkopfs (1, 51, 101, 151, 201) angeordnet sind.

11. Hüftgelenkspacer nach einem der vorangehenden Ansprüche, **dadurch gekennzeichnet, dass**
in dem ersten Verbindungsmittel (8, 58, 108, 158, 208) oder in der Spülflüssigkeits-Einlassöffnung ein erstes Ventilelement (34, 84, 134, 184) angeordnet ist, das ein Rückfließen der Spülflüssigkeit in das erste Verbindungsmittel (8, 58, 108, 158, 208) verhindert, und/oder
in dem zweiten Verbindungsmittel (9, 59, 109, 159, 209) oder in der Spülflüssigkeits-Auslassöffnung ein zweites Ventilelement (38, 88, 138, 188) angeordnet ist, das ein Rückfließen der Spülflüssigkeit in das zweite Verbindungsmittel (9, 59, 109, 159, 209) verhindert, wobei
bevorzugt das erste und/oder das zweite Ventilelement (34, 38, 84, 88, 134, 138, 184, 188) ausgewählt sind aus einem Rückschlagventil, einem Kugelventil mit Feder, einem Lippenventil, einem Bunsenventil oder einem Plattenventil.

12. Hüftgelenkspacer nach einem der vorangehenden Ansprüche, **dadurch gekennzeichnet, dass**
in einer ersten Leitung (32, 82, 132, 182) innerhalb des Prothesenkörpers, die die zumindest eine Spülflüssigkeitseintrittsöffnung (16, 18, 66, 68, 116, 118, 168, 216) mit der Spülflüssigkeits-Auslassöffnung flüssigkeitsdurchlässig verbindet, ein erstes Ventil angeordnet ist, das nur durch Anlegen eines Unterdrucks an der Spülflüssigkeits-Auslassöffnung zu öffnen ist und das ein Zurückfließen der Spülflüssigkeit in die erste Leitung (32, 82, 132, 182) verhindert, und/oder
in einer zweiten Leitung (30, 80, 130, 180, 230) innerhalb des Prothesenkörpers, die die zumindest eine Spülflüssigkeitsaustrittsöffnung (14, 20, 64, 70, 114, 120, 170, 214, 220) mit der Spülflüssigkeits-Einlassöffnung flüssigkeitsdurchlässig verbindet, ein zweites Ventil angeordnet ist, das nur durch Anlegen eines Unterdrucks an der Spülflüssigkeits-Einlassöffnung zu öffnen ist und das ein Zurückfließen der Spülflüssigkeit in die zweite Leitung (30, 80, 130, 180, 230) verhindert.

13. Hüftgelenkspacer nach einem der vorangehenden Ansprüche, **dadurch gekennzeichnet, dass**
in dem Prothesenkörper die Spülflüssigkeits-Einlassöffnung, die Spülflüssigkeits-Auslassöffnung, die zumindest eine Spülflüssigkeitsaustrittsöffnung (14, 20, 64, 70, 114, 120, 170, 214, 220), die zumindest eine Spülflüssigkeitseintrittsöffnung (16, 18, 66, 68, 116, 118, 168, 216) sowie die flüssigkeitsdurchlässigen Verbindungen ausgebildet sind, wobei der Prothesenkörper vorzugsweise aus Kunststoff, Metall, Keramik, Glaskeramik, Knochenzement oder einer Kombination daraus gefertigt ist, und/oder
die Spülflüssigkeits-Einlassöffnung und die Spülflüssigkeits-Auslassöffnung in einer lateralen Oberfläche des Halses (3, 53, 103, 203), der distalen Seite des Kugelkopfs (1, 51, 101, 151, 201) oder des Kragens (4, 54, 104, 204), insbesondere der proximalen Seite des Kragens (4, 54, 104, 204), angeordnet sind.

14. Hüftgelenkspacer nach einem der vorangehenden Ansprüche, **dadurch gekennzeichnet, dass**
wenigsten eine Spülflüssigkeitsaustrittsöffnung (14, 64, 114, 214) der zumindest einen Spülflüssigkeitsaustrittsöffnung (14, 20, 64, 70, 114, 120, 170, 214, 220) am Ende des Schafts (5, 55, 105, 205) angeordnet ist und/oder
der Prothesenkörper mehrteilig ist und einen Schaftteil und wenigstens einen Kopfteil aufweist, die über ein Verbindungsmittel (295, 296), insbesondere über eine Schraubverbindung (295, 296), miteinander lösbar verbindbar sind oder lösbar verbunden sind, vorzugsweise der Prothesenkörper aufweisend einen Schaftteil und mehrere Kopfteile, wobei die Kopfteile Kugelköpfe (201) mit unterschiedlichen Durchmessern haben.

## Claims

1. A femoral hip joint spacer for temporary replacement of a part of a hip joint, the hip joint spacer having
a prosthesis body, the prosthesis body having a ball head (1, 51, 101, 151, 201) with a sliding surface (2, 52, 102, 202), a neck (3, 53, 103, 203), which is connected to the ball head (1, 51, 101, 151, 201), a collar (4, 54, 104, 204), which is connected on a proximal side to the neck (3, 53, 103, 203), and a stem (5, 55, 105, 205), which is connected to a distal side of the collar (4, 54, 104, 204), wherein at least one fastening area (6, 7, 56, 57, 106, 206) is arranged on the stem (5, 55, 105, 205) of the prosthesis body,
the hip joint spacer further having a first tubular and liquid-conveying connecting means (8, 58, 108, 158, 208) for feeding a medical irrigation liquid into the prosthesis body, an irrigation liquid inlet opening in a surface of the prosthesis body, wherein the first connecting means (8, 58, 108, 158, 208) is connected or connectable in liquid-conveying manner to the irrigation liquid inlet opening,
at least one irrigation liquid discharge opening (14, 20, 64, 70, 114, 120, 170, 214, 220) which is arranged in the surface of the prosthesis body outside the at least one fastening area (6, 7, 56, 57, 106, 206), wherein the at least one first irrigation liquid discharge opening (14, 20, 64, 70, 114, 120, 170, 214, 220) is connected inside the prosthesis body in liquid-conveying manner to the irrigation liquid inlet opening **characterized in that**
the femoral hip joint spacer further having
a second tubular and liquid-conveying connecting means (9, 59, 109, 159, 209) for draining the irrigation liquid from the prosthesis body,
an irrigation liquid outlet opening in the surface of the prosthesis body, wherein the second connecting means (9, 59, 109, 159, 209) is connected or connectable in liquid-conveying manner to the irrigation liquid outlet opening,
at least one irrigation liquid intake opening (16, 18, 66, 68, 116, 118, 168, 216) which is arranged in the surface of the prosthesis body outside the at least one fastening area (6, 7, 56, 57, 106, 206)
wherein the at least one irrigation liquid intake opening (16, 18, 66, 68, 116, 118, 168, 216) is connected inside the prosthesis body in liquid-conveying manner to the irrigation liquid outlet opening, wherein
the at least one irrigation liquid discharge opening (14, 20, 64, 70, 114, 120, 170, 214, 220) inside the prosthesis body is not connected in a liquid-conveying manner to the irrigation liquid outlet opening and **in that** the at least one irrigation liquid intake opening (16, 18, 66, 68, 116, 118, 168, 216) inside the prosthesis body is not connected in a liquid-conveying manner to the irrigation liquid inlet opening.

2. The hip joint spacer according to Claim 1, **characterized in that**
the at least one fastening area (6, 7, 56, 57, 106, 206) is delimited or
the at least one fastening area (6, 7, 56, 57, 106, 206) is delimited by a peripheral rib (21, 22, 71, 72, 121, 221) extending up out of the surface of the prosthesis body, such that the at least one fastening area (6, 7, 56, 57, 106, 206) is suitable for accommodating bone cement paste within the rib (21, 22, 71, 72, 121, 221), wherein preferably
at least one of the at least one fastening area (6, 7, 56, 57, 106, 206) is regionally delimited by a part of the collar (4, 54, 104, 204).

3. The hip joint spacer according to any one of the preceding claims, **characterized in that**
at least one first irrigation liquid discharge opening (14, 64, 114, 214) and at least one first irrigation liquid intake opening (16, 66, 116, 216) are arranged in the surface of the prosthesis body on the distal side of the collar (4, 54, 104, 204) or on the stem (5, 55, 105, 205) outside the at least one fastening area (6, 7, 56, 57, 106, 206), and
at least one second irrigation liquid discharge opening (20, 70, 120, 170, 220) and at least one second irrigation liquid intake opening (18, 68, 118, 168) are arranged in the surface of the prosthesis body to the side of the collar (4, 54, 104, 204) or on the proximal side of the collar (4, 54, 104, 204) or on the neck (3, 53, 103, 203) or on the ball head (1, 51, 101, 151, 201), wherein
the at least one first irrigation liquid discharge opening (14, 64, 114, 214) and the at least one second irrigation liquid discharge opening (20, 70, 120, 170, 220) are connected inside the prosthesis body in liquid-conveying manner to the irrigation liquid inlet opening, and wherein
the at least one first irrigation liquid intake opening (16, 66, 116, 216) and the at least one second irrigation liquid intake opening (18, 68, 118, 168) are connected inside the prosthesis body in liquid-conveying manner to the irrigation liquid outlet opening.

4. The hip joint spacer according to Claim 3, **characterized in that**
the at least one first irrigation liquid discharge opening (14, 64, 114, 214) and the at least one first irrigation liquid intake opening (16, 66, 116, 216) are spaced from one another and the at least one second irrigation liquid discharge opening (20, 70, 120, 170, 220) and the at least one second irrigation liquid intake opening (18, 68, 118, 168) are spaced from one another, wherein the distance amounts to at least 5 mm, preferably at least 20 mm and particularly preferably at least 30 mm, and/or
the at least one first irrigation liquid discharge opening (14, 64, 114, 214) and the at least one first irrigation liquid intake opening (16, 66, 116, 216) are demarcated from the at least one fastening area (6, 7, 56, 57, 106, 206) by at least one peripheral rib (21, 22, 71, 72, 121, 221) extending up out of the surface of the prosthesis body.

5. The hip joint spacer according to any one of Claims 3 or 4, **characterized in that**
at least one irrigation liquid discharge opening (20, 70, 120, 170, 220) of the at least one second irrigation liquid discharge opening (20, 70, 120, 170, 220) and at least one irrigation liquid intake opening (18, 68, 118, 168) of the at least one second irrigation liquid intake opening (18, 68, 118, 168) are arranged on the ball head (1, 51, 101, 151, 201) next to the sliding surface (2, 52, 102, 202), preferably on the ball head (1, 51, 101, 151, 201) within 5 mm of the sliding surface (2, 52, 102, 202), and/or
at least one of the at least one first irrigation liquid discharge opening (14, 64, 114, 214) is arranged at the end of the stem (5, 55, 105, 205) and at least one of the at least one first irrigation liquid intake opening (16, 66, 116, 216) is arranged on the distal side of the collar (4, 54, 104, 204), at the point of transition from the collar (4, 54, 104, 204) to the stem (5, 55, 105, 205) or on the proximal side of the stem (5, 55, 105, 205).

6. The hip joint spacer according to any one of the preceding claims, **characterized in that** the at least one irrigation liquid discharge opening (14, 20, 64, 70, 114, 120, 170, 214, 220) and the at least one irrigation liquid intake opening (16, 18, 66, 68, 116, 118, 168, 216) are spaced from one another, wherein the distance amounts to at least 5 mm, preferably at least 20 mm and particularly preferably at least 30 mm.

7. The hip joint spacer according to any one of the preceding claims, **characterized in that** the first connecting means (8, 58, 108, 158, 208) on the side remote from the connection with the irrigation liquid inlet opening and the second connecting means (9, 59, 109, 159, 209) on the side remote from the connection with the irrigation liquid outlet opening in each case have an adapter (24, 25, 74, 75, 124, 125, 174, 175, 224, 225), in particular in each case a Luer Lock adapter (24, 25, 74, 75, 124, 125, 174, 175, 224, 225), and/or
a self-sealing coupling (44, 94, 144, 194) is arranged at the irrigation liquid inlet opening inside the prosthesis body or at the surface of the prosthesis body and a self-sealing coupling (42, 92, 142, 192) is arranged at the irrigation liquid outlet opening inside the prosthesis body or at the surface of the prosthesis body, wherein the first connecting means (8, 58, 108, 158, 208) is detachably connected or connectable to the irrigation liquid inlet opening and the second connecting means (9, 59, 109, 159, 209) is detachably connected or connectable to the irrigation liquid outlet opening.

8. The hip joint spacer according to any one of the preceding claims, **characterized in that** the irrigation liquid inlet opening is a first irrigation liquid inlet opening and the irrigation liquid outlet opening is a first irrigation liquid outlet opening, wherein a second irrigation liquid inlet opening and a second irrigation liquid outlet opening are additionally provided in the surface of the prosthesis body, wherein a self-sealing coupling (44, 46, 94, 96, 144, 146, 194, 196) is in each case arranged at the first irrigation liquid inlet opening, the second irrigation liquid inlet opening, the first irrigation liquid outlet opening and the second irrigation liquid outlet opening, wherein the first connecting means (8, 58, 108, 158, 208) is detachably connectable in liquid-tight manner to the first irrigation liquid inlet opening and to the second irrigation liquid inlet opening and the second connecting means (9, 59, 109, 159, 209) is detachably connectable in liquid-tight manner to the first irrigation liquid outlet opening and to the second irrigation liquid outlet opening and wherein
the first irrigation liquid inlet opening and the second irrigation liquid inlet opening are connected with one another in liquid-conveying manner in the prosthesis body and the first irrigation liquid outlet opening and the second irrigation liquid outlet opening are connected with one another in liquid-conveying manner in the prosthesis body.

9. The hip joint spacer according to any one of the preceding claims, **characterized in that** the sum of the cross-sectional areas of all of the at least one irrigation liquid intake opening (16, 18, 66, 68, 116, 118, 168, 216) together is at least as great as the cross-sectional area of the irrigation liquid inlet opening and/or
the sum of the cross-sectional areas of all of the at least one irrigation liquid discharge opening (14, 20, 64, 70, 114, 120, 170, 214, 220) is at least as great as the cross-sectional area of the irrigation liquid outlet opening.

10. The hip joint spacer according to any one of the preceding claims, **characterized in that** the at least one irrigation liquid discharge opening (14, 20, 64, 70, 114, 120, 170, 214, 220) and the at least one irrigation liquid intake opening (16, 18, 66, 68, 116, 118, 168, 216) are not arranged in the sliding surface (2, 52, 102, 202) of the ball head (1, 51, 101, 151, 201).

11. The hip joint spacer according to any one of the preceding claims, **characterized in that** a first valve element (34, 84, 134, 184) is arranged in the first connecting means (8, 58, 108, 158, 208) or in the irrigation liquid inlet opening, said valve element preventing backflow of the irrigation liquid into the first connecting means (8, 58, 108, 158, 208), and/or
a second valve element (38, 88, 138, 188) is arranged in the second connecting means (9, 59, 109, 159, 209) or in the irrigation liquid outlet opening, said valve element preventing backflow of the irrigation liquid into the second connecting means (9, 59, 109, 159, 209), wherein
the first and/or the second valve elements (34, 38, 84, 88, 134, 138, 184, 188) are preferably selected from a non-return valve, a ball valve with spring, a lip valve, a Bunsen valve or a plate valve.

12. The hip joint spacer according to any one of the preceding claims, **characterized in that** a first valve is arranged in a first duct (32, 82, 132, 182) inside the prosthesis body which connects the at least one irrigation liquid intake opening (16, 18, 66, 68, 116, 118, 168, 216) in liquid-conveying manner to the irrigation liquid outlet opening, said first valve being openable solely by applying a vacuum at the irrigation liquid outlet opening and preventing backflow of the irrigation liquid into the first duct (32, 82, 132, 182), and/or
a second valve is arranged in a second duct (30, 80, 130, 180, 230) inside the prosthesis body which connects the at least one irrigation liquid discharge opening (14, 20, 64, 70, 114, 120, 170, 214, 220) in liquid-conveying manner to the irrigation liquid inlet opening, said second valve being openable solely by applying a vacuum at the irrigation liquid inlet opening and preventing backflow of the irrigation liquid into the second duct (30, 80, 130, 180, 230).

13. The hip joint spacer according to any one of the preceding claims, **characterized in that** the irrigation liquid inlet opening, the irrigation liquid outlet opening, the at least one irrigation liquid discharge opening (14, 20, 64, 70, 114, 120, 170, 214, 220), the at least one irrigation liquid intake opening (16, 18, 66, 68, 116, 118, 168, 216) and the liquid-conveying connections are formed in the prosthesis body, wherein the prosthesis body is preferably made of plastics, metal, ceramics, glass ceramic, bone cement or a combination thereof, and/or
the irrigation liquid inlet opening and the irrigation liquid outlet opening are arranged in a lateral surface of the neck (3, 53, 103, 203), in the distal side of the ball head (1, 51, 101, 151, 201) or of the collar (4, 54, 104, 204), in particular in the proximal side of the collar (4, 54, 104, 204).

14. The hip joint spacer according to any one of the preceding claims, **characterized in that** at least one irrigation liquid discharge opening (14, 64, 114, 214) of the at least one irrigation liquid discharge opening (14, 20, 64, 70, 114, 120, 170, 214, 220) is arranged at the end of the stem (5, 55, 105, 205), and/or
the prosthesis body is of multipart configuration and has a stem part and at least one head part, which are detachably connectable or detachably connected with one another via a connecting means (295, 296), in particular via a screw fastening (295, 296), the prosthesis body preferably having a stem part and a plurality of head parts, wherein the head parts have ball heads (201) of different diameters.

## Revendications

1. Entretoise fémorale d'articulation de hanche permettant de remplacer partiellement une partie de l'articulation de la hanche, l'entretoise d'articulation de hanche présentant
un corps de prothèse, le corps de prothèse présentant une tête sphérique (1, 51, 101, 151, 201) avec une surface de glissement (2, 52, 102, 202), une gorge (3, 53, 103, 203), qui est reliée avec la tête sphérique (1, 51, 101, 201), un col (4, 54, 104, 204), qui est relié avec la gorge (3, 53, 103, 203) au niveau d'un côté proximal, et une tige (5, 55, 105, 205), qui est reliée avec un côté distal du col (4, 54, 104, 204), où au moins une surface de fixation (6, 7, 56, 57, 106, 206) est arrangée sur la tige (5, 55, 105, 205) du corps de prothèse,
l'entretoise d'articulation de hanche présentant en outre un premier système de liaison (8, 58, 108, 158, 208) en forme de tube et laissant passer un liquide pour permettre d'amener un liquide de lavage médicinal dans le corps de prothèse,
un orifice d'entrée de liquide de lavage au niveau d'une surface du corps de prothèse, où le premier système de liaison (8, 58, 108, 158, 208) est relié ou peut être relié en laissant passer le liquide avec l'orifice d'entrée de liquide de lavage,
au moins un orifice d'écoulement de liquide de lavage (14, 20, 64, 70, 114, 120, 170, 214, 220), qui est disposé au niveau de la surface du corps de prothèse à l'extérieur de l'au moins une surface de fixation (6, 7, 56, 57, 106, 206), où l'au moins un orifice d'écoulement de liquide de lavage (14, 20, 64, 70, 114, 120, 170, 214, 220) à l'intérieur du corps de prothèse est relié en laissant passer le liquide avec l'orifice d'entrée de liquide de lavage,
**caractérisée en ce que** l'entretoise fémorale d'articulation de hanche présente en outre un deuxième système de liaison (9, 59, 109, 159, 209) en forme de tube et laissant passer du liquide pour l'évacuation du liquide de lavage hors du corps de prothèse,
un orifice de sortie de liquide de lavage au niveau de la surface du corps de prothèse, où le deuxième système de liaison (9, 59, 109, 159, 209) est relié ou peut être relié avec l'orifice de sortie de liquide de lavage en laissant passer le liquide,
au moins un orifice d'entrée de liquide de lavage (16, 18, 66, 68, 116, 118, 168, 26), qui est disposé au niveau de la surface du corps de prothèse à l'extérieur de l'au moins une surface de fixation (6, 7, 56, 57, 106, 206),
où l'au moins un orifice d'entrée de liquide de lavage (16, 18, 66, 68, 116, 118, 168, 216) à l'intérieur du corps de prothèse est relié avec l'orifice de sortie de liquide de lavage en laissant passer le liquide, où
l'au moins un orifice d'écoulement de liquide de lavage (14, 20, 64, 70, 114, 120, 170, 214, 220) à l'intérieur du corps de prothèse est relié avec l'orifice de sortie de liquide de lavage en ne laissant pas passer le liquide et l'au moins un orifice d'entrée de liquide de lavage (16, 18, 66, 68, 116, 118, 168, 216) à l'intérieur du corps de prothèse est relié avec l'orifice d'entrée de liquide de lavage en ne laissant pas passer de liquide.

2. Entretoise d'articulation de hanche selon la revendication 1, **caractérisée en ce que**
l'au moins une surface de fixation (6, 7, 56, 57, 106, 206) a des limites ou l'au moins une surface de fixation (6, 7, 56, 57, 106, 206) est délimitée avec une collerette (21, 22, 71, 72, 121, 221) périphérique, s'élevant à partir de la surface du corps de prothèse, de sorte que l'au moins une surface de fixation (6, 7, 56, 57, 106, 206) est appropriée pour l'admission d'une pâte de ciment osseux à l'intérieur de la collerette (21, 22, 71, 72, 121, 221), où, de préférence,
au moins une surface parmi l'au moins une surface de fixation (6, 7, 56, 57, 106, 206) est délimitée par endroits par une partie du col (4, 54, 104, 204).

3. Entretoise d'articulation de hanche selon l'une des revendications précédentes,
**caractérisée en ce**
**qu'**au moins un premier orifice d'écoulement de liquide de lavage (14, 64, 114, 214) et au moins un premier orifice d'entrée de liquide de lavage (16, 66, 116, 216) sont disposés au niveau de la surface du corps de prothèse sur le côté distal du col (4, 54, 104, 204) ou sur la tige (5, 55, 105, 205) à l'extérieur de l'au moins une surface de fixation (6, 7, 56, 57, 106, 206), et
au moins un deuxième orifice d'écoulement de liquide de lavage (20, 70, 120, 170, 220) et au moins un deuxième orifice d'entrée de liquide de lavage (18, 68, 118, 168) sont disposés au niveau de la surface du corps de prothèse, latéralement sur le col (4, 54, 104, 204), ou sur le côté proximal du col (4, 54, 104, 204), ou sur la gorge (3, 53, 103, 203), ou sur la tête sphérique (1, 51, 101, 151, 201), où
l'au moins un premier orifice d'écoulement de liquide de lavage (14, 64, 114, 214) et l'au moins un deuxième orifice d'écoulement de liquide de lavage (20, 70, 120, 170, 220) à l'intérieur du corps de prothèse sont reliés avec l'orifice d'entrée de liquide de lavage en laissant passer le liquide, et où
l'au moins un premier orifice d'entrée de liquide de lavage (16, 66, 116, 216) et l'au moins un deuxième orifice d'entrée de liquide de lavage (18, 68, 118, 168) à l'intérieur du corps de prothèse sont reliés avec l'orifice de sortie de liquide de lavage en laissant passer le liquide.

4. Entretoise d'articulation de hanche selon la revendication 3, **caractérisée en ce que**
l'au moins un premier orifice d'écoulement de liquide de lavage (14, 64, 114, 214) et l'au moins un premier orifice d'entrée de liquide de lavage (16, 66, 116, 216) sont espacés l'un de l'autre et l'au moins un deuxième orifice d'écoulement de liquide de lavage (20, 70, 120, 170, 220) et l'au moins un deuxième orifice d'entrée de liquide de lavage (18, 68, 118, 168) sont espacés l'un de l'autre, où l'intervalle est d'au moins 5 mm, de préférence, d'au moins 20 mm, et de manière particulièrement préférée, d'au moins 30 mm, et/ou
l'au moins un premier orifice d'écoulement de liquide de lavage (14, 64, 114, 214) et l'au moins un premier orifice d'entrée de liquide de lavage (16, 66, 116, 216) sont délimités par au moins une collerette (21, 22, 71, 72, 121, 221) périphérique et qui s'élève à partir de la surface du corps de prothèse à partir de l'au moins une surface de fixation (6, 7, 56, 57, 106, 206).

5. Entretoise d'articulation de hanche selon l'une des revendications 3 ou 4, **caractérisée en ce**
**qu'**au moins un orifice d'écoulement de liquide de lavage (20, 70, 120, 170, 220) parmi l'au moins un deuxième orifice d'écoulement de liquide de lavage (20, 70, 120, 170, 220) et l'au moins un orifice d'entrée de liquide de lavage (18, 68, 118, 168) parmi l'au moins un deuxième orifice d'entrée de liquide de lavage (18, 68, 118, 168) sont disposés sur la tête sphérique (1, 51, 101, 151, 201) à côté de la surface de glissement (2, 52, 102, 202), sont disposés sur la tête sphérique (1, 51, 101, 151, 201) de préférence dans un intervalle de 5 mm à côté de la surface de glissement (2, 52, 102, 202), et/ou
au moins un orifice parmi l'au moins un premier orifice d'écoulement de liquide de lavage (14, 64, 114, 214) est disposé à l'extrémité de la tige (5, 55, 105, 205) et au moins un orifice parmi l'au moins un premier orifice d'entrée de liquide de lavage (16, 66, 116, 216) est disposé sur le côté distal du col (4, 54, 104, 204), au niveau de la transition du col (4, 54, 104, 204) vers la tige (5, 55, 105, 205), ou sur le côté proximal de la tige (5, 55, 105, 205).

6. Entretoise d'articulation de hanche selon l'une des revendications précédentes,
**caractérisée en ce que**
l'au moins un orifice d'écoulement de liquide de lavage (14, 20, 64, 70, 114, 120, 170, 214, 220) et l'au moins un orifice d'entrée de liquide de lavage (16, 18, 66, 68, 116, 118, 168, 216) sont espacés l'un de l'autre, où l'intervalle est d'au moins 5 mm, de préférence, d'au moins 20 mm, et de manière particulièrement préférée, d'au moins 30 mm.

7. Entretoise d'articulation de hanche selon l'une des revendications précédentes,
**caractérisée en ce que**
le premier système de liaison (8, 58, 108, 158, 208) sur le côté opposé à la liaison avec l'orifice d'entrée de liquide de lavage et le deuxième système de liaison (9, 59, 109, 159, 209) sur le côté opposé à la liaison avec l'orifice de sortie de liquide de lavage présentent respectivement un adaptateur (24, 25, 74, 75, 124, 125, 174, 175, 224, 225), notamment respectivement un adaptateur type Luer-Lock (24, 25, 74, 75, 124, 125, 174, 175, 224, 225), et/ou
un coupleur auto-obturant (44, 94, 144, 194) est disposé sur l'orifice d'entrée de liquide de lavage à l'intérieur du corps de prothèse ou sur la surface du corps de prothèse et un coupleur auto-obturant (42, 92, 142, 192) est disposé sur l'orifice de sortie de liquide de lavage à l'intérieur du corps de prothèse ou sur la surface du corps de prothèse, où le premier système de liaison (8, 58, 108, 158, 208) est relié ou peut être relié de manière amovible avec l'orifice d'entrée de liquide de lavage et le deuxième système de liaison (9, 59, 109, 159, 209) est relié ou peut être relié de manière amovible avec l'orifice de sortie de liquide de lavage.

8. Entretoise d'articulation de hanche selon l'une des revendications précédentes,
**caractérisée en ce que**
l'orifice d'entrée de liquide de lavage est un premier orifice d'entrée de liquide de lavage et l'orifice de sortie de liquide de lavage est un premier orifice de sortie de liquide de lavage, où, en outre, un deuxième orifice d'entrée de liquide de lavage et un deuxième orifice de sortie de liquide de lavage sont prévus dans la surface du corps de prothèse, où un coupleur auto-obturant (44, 46, 94, 96, 144, 146, 194, 196) est respectivement disposé sur le premier orifice d'entrée de liquide de lavage, le deuxième orifice d'entrée de liquide de lavage, le premier orifice de sortie de liquide de lavage et le deuxième orifice de sortie de liquide de lavage, où le premier système de liaison (8, 58, 108, 158, 208) est étanche pour les liquides et peut être relié de manière amovible avec le premier orifice d'entrée de liquide de lavage et avec le deuxième orifice d'entrée de liquide de lavage et le deuxième système de liaison (9, 59, 109, 159, 209) peut être relié de manière étanche aux liquides et amovible avec le premier orifice de sortie de liquide de lavage et avec le deuxième orifice de sortie de liquide de lavage, et où
le premier orifice d'entrée de liquide de lavage et le deuxième orifice d'entrée de liquide de lavage sont reliés l'un à l'autre dans le corps de prothèse en laissant passer le liquide et le premier orifice de sortie de liquide de lavage et le deuxième orifice de sortie de liquide de lavage sont reliés l'un à l'autre dans le corps de prothèse en laissant passer le liquide.

9. Entretoise d'articulation de hanche selon l'une des revendications précédentes,
**caractérisée en ce que**
la somme des surfaces transversales de tous les orifices parmi les au moins un orifice d'entrée de liquide de lavage (16, 18, 66, 68, 116, 118, 168, 216) ensemble est au moins aussi grande que la surface transversale de l'orifice d'entrée de liquide de lavage, et/ou
la somme des surfaces transversales de tous les orifices parmi au moins un orifice d'écoulement de liquide de lavage (14, 20, 64, 70, 114, 120, 170, 214, 220) est au moins aussi grande que la surface transversale de l'orifice de sortie de liquide de lavage.

10. Entretoise d'articulation de hanche selon l'une des revendications précédentes,
**caractérisée en ce que**
l'au moins un orifice d'écoulement de liquide de lavage (14, 20, 64, 70, 114, 120, 170, 214, 220) et l'au moins un orifice d'entrée de liquide de lavage (16, 18, 66, 68, 116, 118, 168, 216) ne sont pas disposés dans la surface de glissement (2, 52, 102, 202) de la tête sphérique (1, 51, 101, 151, 201).

11. Entretoise d'articulation de hanche selon l'une des revendications précédentes,
**caractérisée en ce**
**qu'**un premier élément de mise à l'air (34, 84, 134, 184) est disposé dans le premier système de liaison (8, 58, 108, 158, 208) ou dans l'orifice d'entrée de liquide de lavage, qui empêche un reflux du liquide de lavage vers le premier système de liaison (8, 58, 108, 158, 208), et/ou
un deuxième élément de mise à l'air (38, 88, 138, 188) est disposé dans le deuxième système de liaison (9, 59, 109, 159, 209) ou dans l'orifice de sortie de liquide de lavage, qui empêche un reflux du liquide de lavage vers le deuxième système de liaison (9, 59, 109, 159, 209), où
de préférence, le premier, et/ou le deuxième élément de mise à l'air (34, 38, 84, 88, 134, 138, 184, 188) sont choisis parmi un clapet anti-retour, un clapet à bille avec ressort, un clapet à lèvre, une valve type Bunsen ou une soupape à plateau.

12. Entretoise d'articulation de hanche selon l'une des revendications précédentes,
**caractérisée en ce que**
dans une première conduite (32, 82, 132, 182) à l'intérieur du corps de prothèse, qui relie l'au moins un orifice d'entrée de liquide de lavage (16, 18, 66, 68, 116, 118, 168, 216) avec l'orifice de sortie de liquide de lavage en laissant passer le liquide, une première soupape est disposée, qui ne peut être ouverte que par l'application d'une pression négative à l'orifice de sortie de liquide de lavage et qui empêche un reflux du liquide de lavage vers la première conduite (32, 82, 132, 182), et/ou
dans une deuxième conduite (30, 80, 130, 180, 230) à l'intérieur du corps de prothèse, qui relie l'au moins un orifice d'écoulement de liquide de lavage (14, 20, 64, 70, 114, 120, 170, 214, 220) avec l'orifice d'entrée de liquide de lavage en laissant passer le liquide, une deuxième soupape est disposée, qui ne peut être ouverte que par l'application d'une pression négative à l'orifice d'entrée de liquide de lavage et qui empêche un reflux du liquide de lavage vers la deuxième conduite (30, 80, 130, 180, 230).

13. Entretoise d'articulation de hanche selon l'une des revendications précédentes,
**caractérisée en ce que**
l'orifice d'entrée de liquide de lavage, l'orifice de sortie de liquide de lavage, l'au moins un orifice d'écoulement de liquide de lavage (14, 20, 64, 70, 114, 120, 170, 214, 220), l'au moins un orifice d'entrée de liquide de lavage (16, 18, 66, 68, 116, 118, 168, 216), ainsi que les systèmes de liaison laissant passer le liquide sont formés dans le corps de prothèse, où le corps de prothèse est fabriqué à base d'une matière plastique, d'un métal, d'une céramique, d'une céramique vitreuse, d'un ciment osseux ou d'une combinaison de ceux-ci, et/ou
l'orifice d'entrée de liquide de lavage et l'orifice de sortie de liquide de lavage sont disposés dans une surface latérale de la gorge (3, 53, 103, 203), du côté distal de la tête sphérique (1, 51, 101, 151, 201) ou du col (4, 54, 104, 204), notamment du côté proximal du col (4, 54, 104, 204).

14. Entretoise d'articulation de hanche selon l'une des revendications précédentes,
**caractérisée en ce**
**qu'**au moins un orifice d'écoulement de liquide de lavage (14, 64, 114, 214) parmi l'au moins un orifice d'écoulement de liquide de lavage (14, 20, 64, 70, 114, 120, 170, 214, 220) est disposé à l'extrémité de la tige (5, 55, 105, 205), et/ou
le corps de prothèse est en plusieurs parties et présente une partie de tige et au moins une partie de tête, qui peuvent être reliées l'une à l'autre de manière amovible ou sont reliées de manière amovible par le biais d'un système de liaison (295, 296), notamment par le biais d'une liaison par vissage (295, 296), de préférence, le corps de prothèse présentant une partie de tige et plusieurs parties de tête, où les parties de tête ont des têtes sphériques (201) avec des diamètres variables.
